# EUROPEAN PATENT APPLICATION

(11) **EP 2 565 650 A1**
(43) Date of publication of application: **06.03.2013**
(21) Application number: 11774666.9
(22) Date of filing: 02.02.2011
(51) Int. Cl.: G01N 35/08, G01N 37/00, C12Q 1/68

(54) **DEVICE AND METHOD FOR MANIPULATING DROPLETS USING GEL-STATE MEDIUM**

(30) Priority: 28.04.2010 JP 2010104581
(71) Applicant: Shimadzu Corporation, Kyoto 604-8511 (JP)
(72) Inventor: OHASHI, Tetsuo, Kyoto-shi Kyoto 604-8511 (JP)
(74) Representative: Kilian Kilian & Partner
(86) International application number: PCT/JP2011/052137
(87) International publication number: WO 2011/135881

(57) **Abstract**

The present invention provides a droplet manipulation method capable of manipulating a droplet only by magnetic-field manipulation without physical manipulation such as electric-field manipulation, and a droplet manipulation device with which such a method can be implemented. A droplet manipulation device for transporting a droplet in a droplet encapsulating medium, comprising: a container 4 which holds the droplet encapsulating medium; a droplet 12, 13, 14 composed of a water-based liquid; a gel-state droplet encapsulating medium 31 which is insoluble or poorly soluble in the water-based liquid; magnetic particles 8 included in the droplet composed of the water-based liquid; and means for applying a magnetic field to generate a magnetic field 61 to transport the droplet together with the magnetic particles. A method for manipulating a droplet in a droplet encapsulating medium held in a container, using the devise.

## Description

### TECHNICAL FIELD

The present invention relates to a device and a method for droplet manipulation using a gel-state medium. That is, the present invention relates to a microdevice and a method for droplet manipulation in the microdevice. More specifically, the present invention relates to a method by which extraction and purification of nucleic acid and gene amplification can be performed in a microdevice.

### BACKGROUND ART

As a standard method for extracting nucleic acid from a biological sample and purifying the nucleic acid, a phenol-chloroform method is conventionally used. However, this method involves complicated operations, uses harmful reagents, and requires high cost of waste liquid treatment, and is therefore becoming less used in other than basic research fields. For example, as a method for purifying nucleic acid for the purpose of genetic testing, a method that utilizes the property of nucleic acid to specifically adsorb to silica is used to easily extract and purify nucleic acid without using harmful reagents. Particularly, a purification method using magnetic silica particles is advantageous for automation, and is therefore applied to nucleic acid extraction and purification devices commercially available from various companies. Such a device makes it possible to obtain a purified nucleic acid sample by performing the step of lysing a sample with a chemical reagent to release nucleic acid and then adding magnetic silica particles thereto to specifically adsorb the nucleic acid to a silica surface; the step of cleaning; and the step of collecting only the nucleic acid. In genetic testing, nucleic acid collected using such a device is used to perform a gene amplification method typified by PCR (Polymerase Chain Reaction).

In recent years, microdevices have been actively developed which are designed to perform all of extraction and purification of nucleic acid and gene amplification typified by PCR on a chip. Generally, microdevices designed to perform extraction and purification of nucleic acid on a micro-scale have been developed in which miniaturized flow channel, pump, valve, etc. are constructed without changing the structure of a nucleic acid extraction and purification device. However, since microdevices for genetic testing are required to be disposable in principle, practical genetic test chips have not become widely used due to the issue of production cost of the devices.

JP-A-2008-12490 (Patent Document 1) discloses a microdevice in which various reactions can be performed on a micro-scale by manipulating a droplet encapsulated in an oil without constructing a pump, a valve, etc. therein. The droplet contains magnetic particles, which makes it possible to manipulate the droplet by a means for applying a magnetic field. The microdevice disclosed in JP-A-2008-12490 uses, as a medium for encapsulating a droplet, an oil having a melting point near ordinary temperature (15°C to 25°C), and therefore can be moved, transported, and stored at ordinary temperature or lower because the oil is solidified.

### ART DOCUMENT PRIOR TO THE APPLICATION

### PATENT DOCUMENT

Patent Document 1: JP-A-2008-12490

### DISCLOSURE OF THE INVENTION

### PROBLEM TO BE SOLVED BY THE INVENTION

According to the method disclosed in JP-A-2008-12490, the device must be heated when used to remelt the solidified oil. Further, the manipulation of droplet transfer is performed in the melted oil, and therefore when a small droplet is separated from the encapsulated droplet regarded as a main droplet, the main droplet needs to be fixed by additionally utilizing an adsorption force produced by an electric field to prevent the main droplet from moving by the manipulation of droplet transfer. For this reason, electric-field control needs to be performed. Therefore, a device for implementing the method disclosed in JP-A-2008-12490 needs to have a means for generating an electric field, which complicates the structure of the device.

Accordingly, it is an object of the present invention to provide a droplet manipulation method capable of manipulating a droplet only by magnetic-field manipulation without physical manipulation such as electric-field manipulation, and a droplet manipulation device with which such a method can be implemented.

### MEANS FOR SOLVING THE PROBLEM

The present inventors have found that the object of the present invention can be achieved by using a gelled droplet encapsulating medium for droplet manipulation performed in a droplet encapsulating medium. This finding has led to the completion of the present invention.

The present invention includes the following inventions.
The following invention is directed to a droplet manipulation device.
(1) A droplet manipulation device for transporting a droplet in a droplet encapsulating medium, comprising:
   a container which holds the droplet encapsulating medium;
   a droplet composed of a water-based liquid;
   a gel-state droplet encapsulating medium which is insoluble or poorly soluble in the water-based liquid;
   magnetic particles included in the droplet composed of the water-based liquid; and
   means for applying a magnetic field to generate a magnetic field to transport the droplet together with the magnetic particles.

The droplet encapsulating medium used in the present invention is a medium capable of encapsulating the water-based liquid in a droplet state.
The phrase "insoluble or poorly soluble in the water-based liquid" means that solubility in the water-based liquid at 25°C is about 100 ppm or less.
The magnetic-field applying means of the droplet manipulation device may be one which can be moved approximately parallel to a transport surface or one composed of two or more magnetic-field applying means arranged approximately parallel to a transport surface.

The following invention is directed to an embodiment in which materials for the gel-state droplet encapsulating medium are specified.
(2) The device according to the above (1), wherein the gel-state droplet encapsulating medium is prepared by mixing a water-insoluble or poorly water-soluble liquid material, and a gelling agent selected from the group consisting of hydroxy fatty acids, dextrin fatty acid esters, and glycerin fatty acid esters.

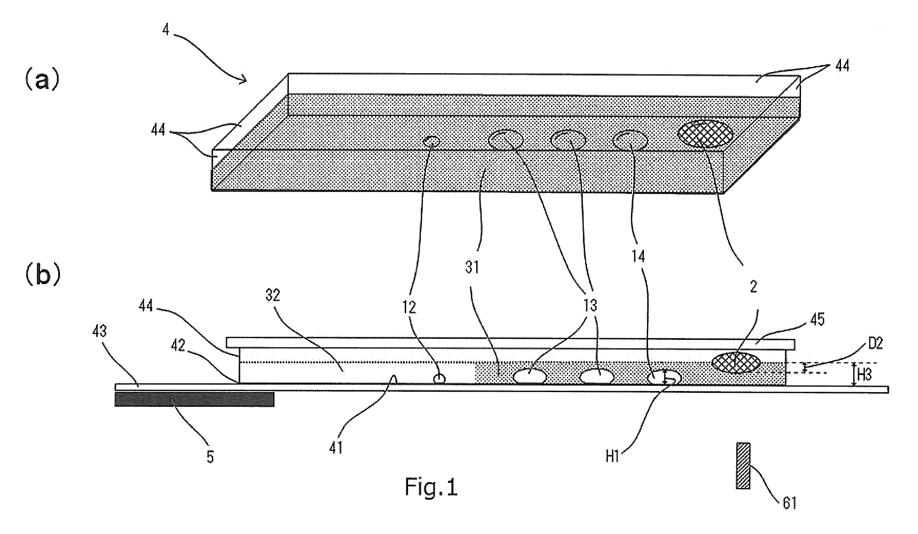

(3) The device according to the above (1) or (2), wherein an another droplet is placed in a path for transporting the droplet.
   Examples of the another droplet in the above-mentioned method are shown in Fig. 1(a) as a droplet composed of a reaction liquid for PCR, a droplet composed of a cleaning liquid, and a droplet composed of a nucleic acid extraction liquid which are encapsulated in a gel-state droplet encapsulating medium.
(4) The device according to any one of the above (1) to (3), wherein the path for transporting the droplet has a temperature gradient.

The following invention is directed to a droplet manipulation method.
(5) A method for manipulating a droplet in a droplet encapsulating medium,
   wherein the droplet encapsulating medium is held in a container,
   the droplet is composed of a water-based liquid including magnetic particles, and
   the droplet encapsulating medium is in a gel state at least before start of droplet manipulation, and is insoluble or poorly soluble in the water-based liquid when the medium is in gel and sol states;
   the method comprising the step of, during the droplet manipulation, transporting the droplet together with the magnetic particles by generating a magnetic field by means for applying a magnetic field.

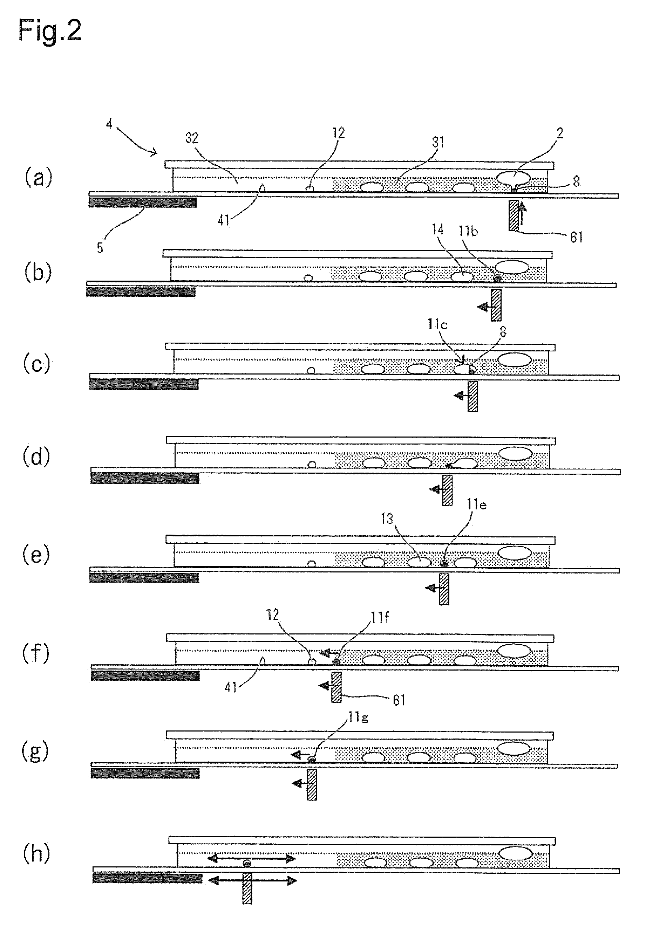

One example of the droplet transport according to the above-mentioned method is shown in Fig. 2(b), 2(e), 2(f), 2(g), or 2(h).
In the above-mentioned method, the droplet encapsulating medium surrounding the droplet that is being transferred after the start of droplet manipulation may be in a gel state (see, for example, Fig. 2(b) or 2(e)) or in a sol state (see, for example, Fig. 2(g) or 2(h)).

The following invention is directed to an embodiment in which a method for encapsulating a droplet is specified.
(6) The method according to the above (5), wherein, before start of the droplet manipulation, a container containing a mixture of a water-insoluble or poorly water-soluble liquid material and a gelling agent is prepared, a droplet is added to the mixture, and then the mixture is turned into a gel to encapsulate the droplet in a gel-state droplet encapsulating medium.

As another method for encapsulating a droplet in the above-mentioned embodiment, droplet encapsulation may be performed by once turning a gelled droplet encapsulating medium into a sol state and adding a droplet thereto; or droplet encapsulation may be performed by directly injecting a droplet into a gel-state droplet encapsulating medium by puncture.

(7) The method according to the above (5) or (6), wherein the droplet is one separated from an another droplet, which includes the magnetic particles and is encapsulated in the gel- or sol-state droplet encapsulating medium in a path for transporting the droplet in the same container, by applying the magnetic field to the another droplet and transferring the droplet along the path for transporting the droplet.
One example of the droplet separation according to the above-mentioned method is schematically shown in Figs. 2 (c) to 2(e).

(8) The method according to the above (5) or (6), wherein the droplet is one separated from an another droplet, which includes the magnetic particles and is placed on the gel-state droplet encapsulating medium in the same container, by generating the magnetic field to the another droplet.
One example of the another droplet placed on the droplet encapsulating medium in the above-mentioned method is shown in Figs. 1(a) and 1(b) as a droplet having a cross-hatched pattern.
One example of the droplet separation according to the above-mentioned method is schematically shown in Figs. 2 (a) to 2(b).

(9) The method according to any one of the above (5) to (8), wherein the droplet is transferred in the gel- or sol-state droplet encapsulating medium and thereby is coalesced with an another droplet encapsulated in the droplet encapsulating medium in a path for transporting the droplet in the same container.
One example of the droplet coalescence according to the above-mentioned method is schematically shown in Figs. 2 (b) and 2(c) or Figs. 2(f) to 2(g).

(10) The method according to any one of the above (5) to (9), wherein a path for transporting the droplet has a temperature gradient.
As one example of a means for creating the temperature gradient in the above-mentioned method, as shown in Fig. 1(b), a ceramic plate and a heater provided so as to be in contact with one end of the ceramic plate can be mentioned.

(11) The method according to the above (10), wherein the droplet encapsulating medium has, in the same container, both a sol phase formed on a high-temperature side of the temperature gradient and a gel phase formed on a low-temperature side of the temperature gradient.
An example of the coexistence of the gel phase and the sol phase according to the above-mentioned method is schematically shown in Figs. 1(b) and 2. In Fig. 2(f), a point where a droplet, which includes magnetic particles and is being displaced, is located has a temperature corresponding to a sol-gel transition point, and therefore the droplet encapsulating medium that is in contact with a transport surface located on the high-temperature side (i.e., on the side closer to the heater) of the point forms a sol phase, and the droplet encapsulating medium that is in contact with a transport surface located on the low-temperature side (i.e., on the side farther from the heater) of the point forms a gel phase.

The following invention is directed to an embodiment in which the magnetic particles and a component adsorbed thereto are cleaned by droplet manipulation.
(12) The method according to any one of the above (9) to (11), wherein the another droplet is composed of a cleaning liquid and the magnetic particles and a component adsorbed thereto are cleaned by the coalescence.

An example of the cleaning according to the above-mentioned method is shown in Figs. 2(b) to 2(c) in which a droplet shown in Fig. 2 (c) into which the aggregated magnetic particles enter is composed of a cleaning liquid.

The following invention is directed to an embodiment in which nucleic acid derived from a biological sample is treated by droplet manipulation.
(13) The method according to any one of the above (8) to (12), wherein a cell lysate and a biological sample are contained in the another droplet to adsorb nucleic acid derived from the biological sample to the magnetic particles.

The following is directed to an embodiment in which a nucleic acid amplification reaction is performed by droplet manipulation.
(14) The method according to any one of the above (11) to (13), wherein the another droplet is composed of a nucleic acid amplification reaction liquid, and
   wherein, in the sol-state droplet encapsulating medium, a droplet composed of a reaction mixture obtained by the coalescence is transferred to a point, which is located on the path for transporting the droplet having the temperature gradient and has a temperature at which a nucleic acid synthesis reaction starts and keeps going, to control a temperature of the reaction mixture.
An example of the temperature control according to the above-mentioned method is shown in Figs. 2(g) to 2(h).

The following is directed to an embodiment in which a nucleic acid amplification reaction is performed by droplet manipulation and further an amplified product is detected by fluorescence detection.
(15) The method according to the above (14), wherein at start of the nucleic acid synthesis reaction, a fluorochrome is included in at least the droplet encapsulating medium out of the droplet composed of the reaction mixture and the droplet encapsulating medium.
One effect obtained by the above-mentioned method is that fluorescence detection based on the amplified product can be performed until the end of the nucleic acid synthesis reaction.

The following invention is directed to a kit for preparing the above-mentioned droplet manipulation device.
(16) A kit for preparing the device according to any one of the above (1) to (4), comprising:
   a container which holds the droplet encapsulating medium;
   the gel-state droplet encapsulating medium, or a water-insoluble or poorly water-soluble liquid material and a gelling agent which are materials for preparing the gel-state droplet encapsulating medium;
   magnetic particles; and
   means for applying a magnetic field.

The above-mentioned kit may be provided in a state where a droplet is encapsulated in the gel-state droplet encapsulating medium. The kit in such an embodiment may be provided in a state where the magnetic particles are contained in the encapsulated droplet.

### EFFECTS OF THE INVENTION

According to the present invention, it is possible to provide a droplet manipulation method capable of manipulating a droplet only by magnetic-field manipulation without physical manipulation such as electric-field manipulation, and a droplet manipulation device with which such a method can be implemented.
Particularly, from a droplet containing magnetic particles and encapsulated in a gel-state droplet encapsulating medium, a trace amount of droplet can be very easily separated together with the magnetic particles. On the other hand, the droplet itself containing the magnetic particles and encapsulated in the droplet encapsulating medium can be easily transferred by turning the droplet encapsulating medium into a sol state. According to the present invention, the gel-state droplet encapsulating medium and the sol-state droplet encapsulating medium can easily coexist in the same container, which makes it very easy to perform a series of operations involving droplet separation and droplet transfer in a closed state. For example, when the present invention is applied to a series of operations of handling a nucleic acid-containing sample, it is possible to perform extraction of nucleic acid from the nucleic acid-containing sample, purification of the nucleic acid, and PCR in one simple device.

### BRIEF DESCRIPTION OF THE DRAWINGS

[Fig.1] Fig.1(a) is a perspective view of a container having a droplet encapsulating medium 31 filled therein, in which droplets (each of which is composed of a nucleic acid extraction liquid 14, a cleaning liquid 13, or a reaction liquid 12 for PCR) are encapsulated in the droplet encapsulating medium 31 and a droplet 2 composed of a nucleic acid-containing sample containing magnetic particles dispersed therein is placed on the droplet encapsulating medium 31; and Fig. 1(b) is a sectional view of the container shown in Fig. 1(a) provided with a cover 45, a substrate (ceramic plate) 43, and a heater 5 to create a temperature gradient.
[Fig.2] Figs.2(a) to 2(h) are schematic views of the container shown in Fig. 1 in which a nucleic acid amplification reaction is performed by sampling the nucleic acid-containing sample from the droplet 2 together with the magnetic particles 8 dispersed in the droplet 2 by manipulation using a magnet 61 (Fig. 2(a)); transferring the sampled nucleic acid-containing sample together with the magnetic particles 8 (Fig. 2(b)); extracting nucleic acid (Fig.2(c)); transferring a sample containing the extracted nucleic acid together with the magnetic particles (Fig. 2(d)); cleaning the sample and the magnetic particles, and coalescing the nucleic acid and the magnetic particles with the nucleic acid amplification reactionliquid 12 (Figs. 2 (e) and 2(f))); and transferring the reaction liquid to a spot having a temperature necessary for nucleic acid amplification (Fig. 2(g)).
[Fig.3] Fig.3 shows photographs taken during a series of operations performed in Example 1 using the device shown in Fig. 2, wherein symbols (a) to (h) and numerals attached to elements in the photographs correspond respectively to those shown in Fig. 2.
[Fig.4] Figs.4(a) and 4(b) show other examples of droplet encapsulation.
[Fig.5] Fig.5 shows the result of electrophoresis performed to detect an amplified product obtained by a nucleic acid amplification reaction performed in Example 1.
[Fig.6] Fig. 6 shows images obtained in Reference Example 1 by observing fluorescence by ultraviolet irradiation after the completion of a PCR reaction when a fluorochrome was added to silicone oil (a-1) and when a fluorochrome was not added (b-1).
[Fig. 7] Fig. 7 is a schematic diagram showing the configuration of equipment for performing PCR by transferring a droplet 11, which is composed of a reaction liquid for PCR containing magnetic particles 8 and is encapsulated in a droplet encapsulating medium 3 filled in a container 4, with the use of a magnet 61 to detect a PCR product in real time during PCR by fluorescence detection.

### DESCRIPTION OF REFERENCE NUMERALS

- 1: encapsulated droplet
- 3: droplet encapsulating medium
- 4: container
- 41: transport surface
- 5: heat source
- 61: magnetic-field applying means
- 8: magnetic particles

### MODES FOR CARRYING OUT THE INVENTION

### [1. Droplet]

A droplet used in the present invention is a liquid lump having a shape (an almost spherical shape or its deformed shape) determined by a balance between a pressure difference between the inside and outside of a droplet comprising a liquid, and a surface tension generated by the intermolecular force of the liquid forming the droplet.

### [1-1. Water-Based Liquid]

A liquid forming the droplet used in the present invention is not particularly limited as long as it is a water-based liquid insoluble or poorly soluble in a droplet encapsulating medium that will be described later, and may be water, an aqueous solution, or an aqueous suspension. The water-based liquid may contain any component to be subjected to a reaction or a treatment to which the present invention can be applied.

Examples of the reaction include a chemical reaction and a biochemical reaction. The chemical reaction may be any reaction performed in a water-based system and involving a chemical change. The change in a substance may be any one of chemical combination, decomposition, oxidation, and reduction. The biochemical reaction may be any reaction involving a change in a biological substance. Examples of such a biochemical reaction include synthesis systems of biological substances such as nucleic acid, proteins, lipids, and sugars, metabolic systems, and immune systems.
The treatment may be any treatment regardless of whether the treatment involves a change in a substance or not. The change in a substance may be either a chemical change or a physical change. Examples of the treatment include pretreatment performed prior to the above-mentioned reaction or an analysis, fractionation (separation), dissolution, mixing, dilution, stirring, and temperature control (heating and cooling).

Specific examples of the water-based liquid include a nucleic acid amplification reaction liquid for performing nucleic acid amplification reaction, a sample containing nucleic acid to be amplified, a nucleic acid extraction liquid for extracting nucleic acid, a magnetic particle cleaning liquid for cleaning nucleic acid, and a nucleic acid releasing liquid for releasing nucleic acid.
Hereinbelow, the liquids mentioned above as specific examples of the water-based liquid, and reactions and treatments to which these liquids are subjected will be further described.

### [1-1-1. Nucleic Acid Amplification Reaction Liquid]

The nucleic acid amplification reaction liquid used in the present invention contains, in addition to various elements usually used in a nucleic acid amplification reaction, at least nucleic acid to be amplified and magnetic particles.

As will be described later, the nucleic acid amplification reaction is not particularly limited, and therefore the various elements used in a nucleic acid amplification reaction can be appropriately determined by those skilled in the art based on, for example, a known nucleic acid amplification method, examples of which will be mentioned later. Usually, a salt such as MgCl₂ or KCl, a primer, deoxyribonucleotides, a nucleic acid synthase, and a pH buffer solution are included. The above-mentioned salt to be used may be appropriately changed to another salt. There is a case where a substance for reducing non-specific priming, such as dimethylsulfoxide, is further added.

A source of the nucleic acid to be amplified is not particularly limited. The nucleic acid to be amplified may be prepared by appropriately performing pretreatment on a separately-prepared sample containing nucleic acid. Examples of the pretreatment include treatments that are unaffected by a fluorochrome contained in the encapsulating medium, such as a treatment for extracting nucleic acid from a nucleic acid-containing sample, a treatment for cleaning magnetic particles to which nucleic acid is adsorbed, and a treatment for releasing nucleic acid from magnetic particles.
The sample containing nucleic acid to be amplified is not particularly limited, and examples thereof include living body-derived samples such as animal and plant tissues, bodily fluids, and excretions; and nucleic acid-containing materials such as cells, protozoa, fungi, bacterium, and viruses. The bodily fluids include blood, spinal fluid, saliva, and milk, and the excretions include feces, urine, and sweat, and they may be used in combination. The cells include white blood cells and platelets contained in blood; and exfoliated mucosal cells such as exfoliated oral mucosal cells and other exfoliated mucosal cells, and they may be used in combination. The nucleic acid-containing sample may be prepared as, for example, a mixture with a cell suspension, a homogenate, or a cell lysate.
It is to be noted that, in the present invention, an example of the nucleic acid-containing sample or a sample obtained by performing pretreatment on the nucleic acid-containing sample is sometimes referred to as a nucleic acid-containing liquid.

The nucleic acid amplification reaction liquid used in the present invention may further contain, in addition to the above-mentioned components, a blocking agent. The blocking agent may be used to prevent deactivation of a nucleic acid polymerase due to adsorption to, for example, the inner wall of a reaction container or the surfaces of the magnetic particles.
Specific examples of the blocking agent include proteins such as bovine serum albumin (namely, BSA), other albumins, gelatin (namely, denatured collagen), casein, and polylysine; and peptides (all of which may be either natural or synthetic).

The nucleic acid amplification reaction to which the present invention is applied is not particularly limited, and examples of a method used to perform the nucleic acid amplification reaction include a PCR method (U.S. Patent Nos. 4683195, 4683202, 4800159, and 4965188), a LCR method (U.S. Patent No. 5494810), a Qβ method (U.S. Patent No. 4786600), a NASBA method (U.S. Patent No. 5409818), a LAMP method (U.S. Patent No. 3313358), an SDA method (U.S. Patent No. 5455166), an RCA method (U.S. Patent No. 5354688), an ICAN method (Japanese Patent No. 3433929), and a TAS method (Japanese Patent No. 2843586).
The composition of the reaction liquid required for the nucleic acid amplification reaction and the reaction temperature can be appropriately selected by those skilled in the art.

In a real-time nucleic acid amplification method, an amplified product is labeled with a fluorochrome that can stain double-stranded DNA, and therefore a change in the fluorochrome can be observed by heating the double-stranded DNA.
Examples of a detecting method used in such a real-time nucleic acid amplification method include the following methods.

For example, when only a desired target can be amplified by a highly specific primer, an intercalator method using, for example, SYBR (Registered trade mark) GREEN I is used.
An intercalator that emits fluorescence when binding to double-stranded DNA binds to double-stranded DNA synthesized by a nucleic acid amplification reaction, and emits fluorescence by irradiation with exciting light. By detecting the intensity of the fluorescence, the amount of amplified product produced can be monitored. This method is not required to design and synthesize a fluorescence-labeled probe specific to a target, and is therefore easily used to measure various targets.

When it is necessary to distinctively detect very similar sequences or SNPs typing is performed, a probe method is used. An example of the probe method is a TaqMan (Registered trade mark) probe method using, as a probe, an oligonucleotide whose 5' end is modified with a fluorescent material and 3' end is modified with a quencher material.
The TaqMan probe is specifically hybridized with template DNA in an annealing step, but even when the fluorescent material is irradiated with exciting light, fluorescence emission is suppressed by the quencher present in the probe. In an extension reaction step, the TaqMan probe hybridized with the template is decomposed by the 5' → 3' exonuclease activity of TaqDNA polymerase so that the fluorochrome is released from the probe, and therefore suppression by the quencher is cancelled and fluorescence is emitted. By measuring the intensity of the fluorescence, the amount of amplified product produced can be monitored.

The principles on which DNA is quantified by real-time PCR by such a method will be described below. First, PCR is performed using, as templates, standard samples of known concentrations prepared by serial dilution to determine threshold cycles (Ct values) at which the amount of amplified product reaches a certain level. The Ct values are plotted along a literal axis and the initial amounts of DNA are plotted along a vertical axis to prepare a calibration curve.
A PCR reaction is performed also on a sample of an unknown concentration under the same conditions to determine a Ct value. The amount of target DNA contained in the sample can be determined from the Ct value and the above-mentioned calibration curve.

The melting curve of the amplified product can also be obtained by further irradiating the amplified product with exciting light from thermal denaturation to annealing.
Double-stranded DNA generated by a nucleic acid amplification reaction has an inherent Tm value depending on DNA length and base sequence. That is, when the temperature of a droplet containing DNA labeled with a fluorochrome is gradually increased, a temperature at which fluorescence intensity rapidly decreases is detected. As a result of examination of the amount of change in fluorescence intensity, a temperature peak thereof is in close agreement with a Tm value defined by the base sequence and length of the DNA. This makes it possible to exclude data observed by generation of not a target gene but, for example, a primer dimer (i.e., false-positive data) from positive data. In genetic testing, a non-specific reaction often occurs due to foreign substances contained in a sample, and therefore exclusion of such false-positive data is important. Further, it is also possible to determine whether or not the amplified product is specific to a target gene.

### [1-1-2. Nucleic Acid Extraction Liquid]

As the nucleic acid extraction liquid used to extract nucleic acid, a buffer solution containing a chaotropic material, EDTA, Tris-HCl, etc. can be mentioned. Examples of the chaotropic material include guanidinium hydrochloride, guanidine isothiocyanate, potassium iodide, urea, and the like.

A specific method for extracting nucleic acid from a nucleic acid-containing sample can be appropriately determined by those skilled in the art. In the present invention, magnetic particles are used to transport nucleic acid in the droplet encapsulating medium, and therefore a nucleic acid extraction method using magnetic particles is preferably used. For example, nucleic acid can be extracted from a nucleic acid-containing sample and purified using magnetic particles with reference to JP-A-2-289596.

### [1-1-3. Cleaning Liquid]

As the cleaning liquid, any cleaning liquid can be used as long as it is a solution that can dissolve components (e.g., proteins and sugars) other than nucleic acid contained in a nucleic acid-containing sample, or components of a reagent or the like used in previously-performed another treatment such as nucleic acid extraction, while allowing nucleic acid to remain adsorbed to the surfaces of magnetic particles. Specific examples of such a cleaning liquid include high-salt concentration aqueous solutions such as sodium chloride, potassium chloride, ammonium sulfate, and the like; and alcohol aqueous solutions such as ethanol, isopropanol, and the like.
A specific method for cleaning the magnetic particles to which nucleic acid is adsorbed can also be appropriately determined by those skilled in the art. The frequency of cleaning of the magnetic particles to which nucleic acid is adsorbed can be appropriately determined by those skilled in the art so that a nucleic acid amplification reaction is not undesirably inhibited. From the same viewpoint, the cleaning step may be omitted.
The number of droplets composed of the cleaning liquid may be at least the same as the frequency of cleaning.

### [1-1-4. Nucleic Acid Releasing Liquid]

As the nucleic acid releasing liquid, water or a buffer solution containing a low concentration of salt can be used. Specific examples of such a nucleic acid releasing liquid include Tris buffer solutions, phosphate buffer solutions, and distilled water.
A specific method for releasing nucleic acid from magnetic particles to which the nucleic acid is adsorbed can also be appropriately determined by those skilled in the art.

### [1-1-5. Other Water-Based Liquids]

The compositions of water-based liquids subjected to any reactions and treatments other than the above-mentioned reactions and treatments can also be easily determined by those skilled in the art.

### [1-2. Amount of Droplet]

The amount of the droplet completely encapsulated in the encapsulating medium may be, for example, 0.1 µL to 10 µL.

### [1-3. Magnetic Particles]

According to a method of the present invention, magnetic particles are included in the droplet so that the droplet can be transferred by moving a magnetic field. The magnetic particles usually have hydrophilic groups on their surfaces. The magnetic particles may be previously encapsulated in the droplet encapsulating medium contained in a container, and in this case, the magnetic particles are preferably contained in the droplet. Alternatively, when a kit for preparing a device according to the present invention is provided, the magnetic particles may be one of items included in the kit separately from a container and a droplet encapsulating medium or its materials.

The magnetic particles are not particularly limited as long as they are particles that respond to magnetism. Examples of such magnetic particles include particles having a magnetic substance such as magnetite, γ-iron oxide, manganese zinc ferrite, and the like. The magnetic particles may have surfaces having a chemical structure that specifically binds to a material to be subjected to the above-mentioned reaction or treatment, such as an amino group, a carboxyl group, an epoxy group, avidin, biotin, digoxigenin, protein A, protein G, a complexed metal ion, or an antibody; or surfaces adapted to specifically bind to the material by electrostatic force or Van der Waals force. This makes it possible to selectively adsorb the material to be subjected to a reaction or a treatment to the surfaces of the magnetic particles.
Examples of the hydrophilic group on the surfaces of the magnetic particles include a hydroxyl group, an amino group, a carboxyl group, a phosphoric group, a sulfonic group, and the like.

The magnetic particles may further comprise, in addition to the above-mentioned elements, various elements appropriately selected by those skilled in the art. Specific preferred examples of the magnetic particles having hydrophilic groups on their surfaces include particles composed of a mixture of a magnetic substance and silica and/or an anion-exchange resin, magnetic particles whose surfaces are covered with silica and/or an anion-exchange resin, magnetic particles whose surfaces are covered with gold to which hydrophilic groups are attached via mercapto groups, and gold particles containing a magnetic substance and having surfaces to which hydrophilic groups are attached via mercapto groups.

The average particle diameter of the magnetic particles whose surfaces have hydrophilic groups may be about 0.1 µm to 500 µm. When the average particle diameter is small, the magnetic particles are likely to be present in a state where the particles are dispersed in the droplet.
As an example of commercially-available magnetic particles, Magnetic Beads provided as a constituent reagent of Plasmid DNA Purification Kit MagExtractor -Plasmid- sold by TOYOBO Co., Ltd. can be mentioned. When magnetic particles such as those sold as a constituent reagent of a kit are used, the magnetic particles are preferably cleaned by suspending an undiluted commercial liquid product containing magnetic particles in pure water (e.g., in pure water whose amount is about ten times greater than that of the undiluted commercial liquid product). After being suspended in pure water, the magnetic particles can be cleaned by removing supernatant by a centrifugal operation. The suspending of the magnetic particles in pure water and removal of supernatant may be repeatedly performed. The cleaned magnetic particles may be used in the present invention in a dispersed state in pure water.

Such magnetic particles are incorporated into the droplet and therefore can be transferred together with the droplet in a direction, in which a means for applying a magnetic field is moved, by fluctuating a magnetic field. This makes it possible to transferred the droplet while the droplet keeps droplet state thereof.

### [2. Droplet Encapsulating Medium]

As the droplet encapsulating medium, a chemically-inactive material insoluble or poorly soluble in the liquid constituting the droplet is used. The chemically-inactive material refers to a material having no chemical influence on the liquid constituting the droplet during various operations such as droplet fractionation (separation), mixing, dissolution, dilution, stirring, heating, and cooling. More specifically, the droplet encapsulating medium used in the present invention is in a gel state at least before droplet manipulation; and is insoluble or poorly soluble in the nucleic acid amplification reaction liquid constituting the droplet in both cases where the medium is in the gel state, and where a temperature of the medium exceeds a sol-gel transition point thereof and the medium is turned into a sol state. In the present invention, a water-insoluble or poorly water-soluble liquid material that can be turned into a gel by adding a gelling agent is usually used.

When a fluorescent material that will be described later is dissolved in the droplet encapsulating medium, a material that can dissolve the fluorescent material can be appropriately selected by those skilled in the art as a material for the droplet encapsulating medium. For example, a material having a phenyl group or the like as a component having a certain level of intramolecular polarity is sometimes preferred. More specifically, a phenyl group-containing silicone oil such as diphenyldimethicone can be used as a material for the droplet encapsulating medium.

When a kit for preparing the device according to the present invention is provided, the liquid material may be previously turned into a gel state by mixing with the gelling agent or the liquid material that has not yet been turned into a gel state and the gelling agent may be prepared as separate items.

### [2-1. Gel-Sol Transition Point]

When the droplet encapsulating medium is exposed to a temperature lower than the gel-sol transition point thereof, the droplet encapsulating medium is turned into a state not having flowability (i.e., a gel state) allowing the transfer of the droplet encapsulated in the droplet encapsulating medium. This makes it possible to fix the droplet at an arbitrary position to prevent the droplet encapsulated in the droplet encapsulating medium from moving in an unexpected direction. Further, it is also possible, while the droplet encapsulated in the droplet encapsulating medium is fixed in such a manner as described above, to easily transfer the magnetic particles contained in the droplet and a material adsorbed to the magnetic particles (more specifically, a material or a liquid that is adsorbed to the surfaces of the magnetic particles, and is to be subjected to a reaction or a treatment). Therefore, even when encapsulated droplets are arranged in positions close to each other, they are not mixed together and therefore magnetic particles and a material adsorbed thereto can be easily moved between these encapsulated droplets.
On the other hand, when the droplet encapsulating medium is exposed to a temperature higher than the gel-sol transition point thereof, the droplet encapsulating medium itself is turned into a state having flowability (i.e., a sol state). This makes it possible to transfer said encapsulated droplet. Even when the volume of the droplet is relatively larger than the total volume of the magnetic particles, the entire droplet can be transferred.
By placing such a droplet encapsulating medium in a temperature variable region that will be described later, as shown in Fig. 1(b), it is possible to easily achieve a state where both a phase of a gel 31 having no flowability and a phase of a sol 32 having no flowability coexist in the same container.

The sol-gel transition point can be set to 40 to 50°C.
The sol-gel transition point may vary depending on conditions such as the type of oil used, the type of gelling agent used, and the amount of gelling agent added. Therefore, such conditions are appropriately selected by those skilled in the art so that a desired sol-gel transition point can be achieved.

### [2-2. Water-Insoluble or Poorly Water Soluble Liquid Material]

As the water-insoluble or poorly water-soluble liquid material, an oil whose solubility in water at 25°C is about 100 ppm or less and which is in a liquid state at an ordinary temperature (20°C ± 15°C) may be used. For example, such an oil may be one or a combination of two or more selected from the group consisting of liquid fats and fatty oils, an ester oil, a hydrocarbon oil, and a silicone oil.

Examples of the liquid fats and fatty oils include linseed oil, camellia oil, macadamia nut oil, corn oil, mink oil, olive oil, avocado oil, sasanqua oil, castor oil, safflower oil, persic oil, cinnamon oil, jojoba oil, grape seed oil, sunflower oil, almond oil, rape oil, sesame oil, wheat germ oil, rice germ oil, rice bran oil, cottonseed oil, soybean oil, peanut oil, tea oil, evening primrose oil, egg-yolk oil, liver oil, coconut oil, palm oil, palm kernel oil, and the like.

Examples of the ester oil include: octanoic acid esters such as cetyl octanoate; lauric acid esters such as hexyl laurate; myristic acid esters such as isopropyl myristate and octyldodecyl myristate; palmitic acid esters such as octyl palmitate; stearic acid esters such as isocetyl stearate; isostearic acid esters such as isopropyl isostearate; isopalmitic acid esters such as octyl isopalmitate; oleic acid esters such as isodecyl oleate; adipic acid esters such as isopropyl adipate; sebacic acid esters such as ethyl sebacate; malic acid esters such as isostearyl malate; glyceryl trioctanoate; glyceryl triisopalmitate, and the like.

Examples of the hydrocarbon oil include pentadecane, hexadecane, octadecane, mineral oil, liquid paraffin, and the like.
Examples of the silicone oil include dimethyl polysiloxane; phenyl group-containing silicone oils such as methyl phenyl polysiloxane and others; methylhydrogen polysiloxane, and the like.

### [2-3. Gelling Agent]

As the gelling agent, one oil gelling agent or a combination of two or more oil gelling agents selected from the group consisting of hydroxy fatty acids, dextrin fatty acid esters, and glycerin fatty acid esters may be used.

The hydroxy fatty acids are not particularly limited as long as they are fatty acids having a hydroxyl group. Specific examples of such hydroxy fatty acids include hydroxymyristic acid, hydroxypalmitic acid, dihydroxypalmitic acid, hydroxystearic acid, dihydroxystearic acid, hydroxymargaric acid, ricinoleic acid, ricinelaidic acid, linolenic acid, and the like. Among them, hydroxystearic acid, dihydroxystearic acid, and ricinoleic acid are preferred. These hydroxy fatty acids may be used singly or in combination of two or more of them. An animal and plant oil fatty acid (e.g., castor oil fatty acid, hydrogenated castor oil fatty acid, or the like) which is a mixture of two or more of the above-mentioned examples may also be used as the hydroxy fatty acid.

Examples of the dextrin fatty acid esters include dextrin myristate (manufactured by Chiba Flour Milling Co., Ltd. under the trade name of "Rheopearl MKL"), dextrin palmitate (manufactured by Chiba Flour Milling Co., Ltd. under the trade name of "Rheopearl KL" or "Rheopearl TL"), and dextrin palmitate/2-ethylhexanoate (manufactured by Chiba Flour Milling Co., Ltd. under the trade name of "Rheopearl TT").

Examples of the glycerin fatty acid esters include glyceryl behenate, glyceryl octastearate, and glyceryl eicosanoate. These glycerin fatty acid esters may be used singly or in combination of two or more of them. Specific examples of the glycerin fatty acid ester include "TAISET 26 (trade name)" (manufactured by Taiyo Kagaku Co., Ltd.) containing 20% glyceryl behenate, 20% glyceryl octastearate, and 60% hardened palm oil, and "TAISET 50 (trade name)" (manufactured by Taiyo Kagaku Co., Ltd.) containing 50% glyceryl behenate and 50% glyceryl octastearate.

The amount of the gelling agent to be added to the water-insoluble or poorly water-soluble liquid material is, for example, 0.1 to 0.5 wt%, 0.5 to 2 wt%, or 1 to 5 wt% of the total weight of the liquid material. However, the amount of the gelling agent to be added is not particularly limited thereto, and can be appropriately determined by those skilled in the art so that a desired gel-sol state can be achieved.

A gelation method can be appropriately determined by those skilled in the art. More specifically, the water-insoluble or poorly water-soluble liquid material is heated, the gelling agent is added to and completely dissolved in the heated liquid material to obtain a solution, and then the solution is cooled. The heating temperature may be appropriately determined in consideration of the physical properties of the liquid material used and the physical properties of the gelling agent used. For example, the heating temperature is sometimes preferably about 60 to 70°C. The dissolution of the gelling agent is preferably performed by gently mixing the liquid material and the gelling agent. The cooling is preferably slowly performed. For example, the cooling may be performed in about 1 to 2 hours. The cooling can be completed by lowering the temperature of the solution to, for example, an ordinary temperature (20°C ± 15°C) or lower, preferably 4°C or lower. As the above-mentioned preferred example of the gelation method, one using the above-mentioned "TAISET 26" (manufactured by Taiyo Kagaku Co., Ltd.) can be mentioned.

### [2-4. Example of Droplet Encapsulating Medium]

An example of the desired gel-sol state is one in which the above-mentioned sol-gel transition point can be achieved.
Another example of the desired gel-sol state is one in which a gel state where a completely-encapsulated droplet can be properly fixed can be achieved. A preferred example of the state where the completely-encapsulated droplet is properly fixed is one in which the encapsulated droplet is not moved by an external force on the order of at least gravity. The phrase "not moved" preferably means that a position where a droplet is in contact with the bottom surface of a container is hardly changed.

Another example of the desired gel-sol state is one in which when, as shown in Fig. 1(b), a droplet 2 of about 0.05 to 5 µL (provided as an aqueous solution or a suspension) containing about 10 to 1000 µg of magnetic particles is placed on a gel-state droplet encapsulating medium 31, and then, as shown in Fig. 2(a), a magnetic field is applied by a magnet 61 from the bottom surface side of a container, magnetic particles 8 contained in the droplet 2 respond to the magnetic field and sink to the bottom surface of the container together with a material adsorbed to the magnetic particles 8.

Another example of the desired gel-sol state is one in which the droplet encapsulating medium in a sol state has a kinetic viscosity of 5 mm²/s to 100 mm²/s, preferably 5 mm²/s to 50 mm²/s, for example, about 20 mm²/s (50°C). Particularly, when a nucleic acid amplification reaction that requires a high temperature condition near 100°C is performed, the droplet encapsulating medium to be used preferably has such a kinetic viscosity. If the kinetic viscosity is less than 5 mm²/s, the droplet encapsulating medium is likely to volatilize at a high temperature, and on the other hand, if the kinetic viscosity exceeds 100 mm²/s, transfer of the droplet achieved by fluctuating a magnetic field is likely to be inhibited. As one of materials preferably used as such a droplet encapsulating medium, one obtained by adding a gelling agent to a silicone oil can be mentioned.

As for the physical properties of the droplet encapsulating medium in a gel state, its storage viscoelasticity E', which is one of dynamic viscoelastic properties, is preferably 10 to 100 kPa, more preferably 20 to 50 kPa at an ordinary temperature (20°C ± 15°C).

### [2-5. Amount of Encapsulating Medium]

The amount of the droplet encapsulating medium used can be determined without any limitation as long as it is enough to completely encapsulate the droplet. The present invention allows the droplet encapsulating medium to be used in such an amount that makes it impossible to adequately detect an amplified product in the case of a conventional method (i.e., a method in which a fluorescent material is added only to a droplet at the start of a nucleic acid amplification reaction).
More specifically, the droplet encapsulating medium can be used in an amount 1,000 to 50,000 times or 20,000 to 200,000 times the volume of the droplet. The use of the droplet encapsulating medium in an amount within the above range is preferred in that the droplet can be transported with high manipulability. If the amount of the droplet encapsulating medium used exceeds the above upper limit, it tends to take a long time to create temperature conditions suitable for the start of PCR to start analysis.

For example, when a nucleic acid amplification reaction is performed in the droplet according to an embodiment of the present invention in which a fluorescent material is contained in at least the droplet encapsulating medium, the present invention allows the droplet encapsulating medium to be used in such an amount that makes it impossible to adequately detect an amplified product in the case of a conventional method in which a fluorescent material is added only to a droplet at the start of a nucleic acid amplification reaction. For example, the droplet encapsulating medium can be used in an amount 1,000 to 10,000 times or 5,000 to 100,000 times the volume of the droplet. If the amount of the droplet encapsulating medium used is less than the above lower limit, the amount of the fluorochrome contained in the droplet is excessive and therefore an S/N ratio tends to lower due to background rise during fluorescence detection. On the other hand, if the amount of the droplet encapsulating medium used exceeds the above upper limit, detection sensitivity tends to lower due to diffusion of the fluorochrome from the droplet.

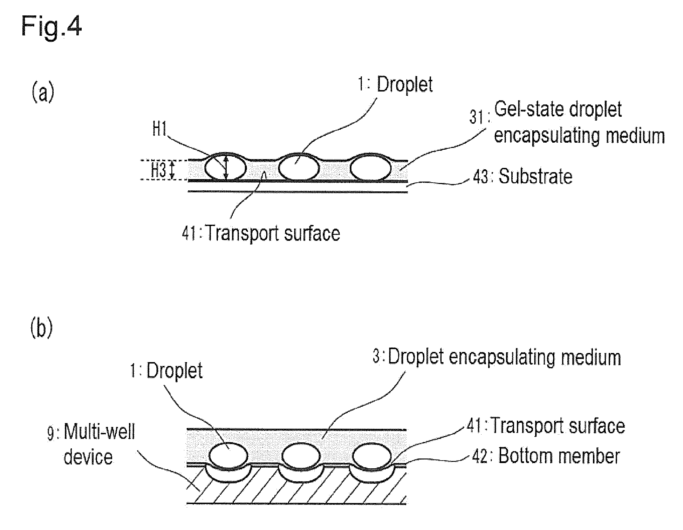

The droplet encapsulating medium is contained in a container. More specifically, as shown in Fig. 1(b), the droplet encapsulating medium is filled in a container so as to come into contact with a transport surface 41. In this case, the filling height (filling thickness) H3 of the droplet encapsulating medium in the container can be determined without any limitation as long as the amount of the droplet encapsulating medium is enough to completely encapsulate the droplet. Usually, the filling height H3 can be made equal to or larger than a height H1 of the droplet encapsulated in the droplet encapsulating medium.
The droplet encapsulating medium used in the present invention has an excellent ability to encapsulate the droplet, and therefore the following embodiment is acceptable. That is, as shown in Fig. 4(a), an embodiment in which a filling height H3 of part of the droplet encapsulating medium where droplets 1 are not present in a container is lower than the height H1 of the encapsulated droplet (which has the largest volume among the encapsulated droplets in the container) is also acceptable.

### [3. Fluorescent Material]

The fluorescent material can be included in at least the droplet encapsulating medium. This embodiment is preferred when a nucleic acid amplification reaction is performed in the droplet. In this case, the fluorescent material needs to be contained in at least the droplet encapsulating medium at the start of the nucleic acid amplification reaction at the latest. It is to be noted that it has already been confirmed by the present inventors that when pretreatment for the nucleic acid amplification reaction is also performed in another droplet in the same droplet encapsulating medium, the fluorescent material does not affect the pretreatment even when the fluorescent material is contained in the droplet encapsulating medium in the stage of the pretreatment.

The fluorescent material is not particularly limited, and one used to detect nucleic acid in a nucleic acid amplification reaction can be appropriately determined by those skilled in the art. Specific examples of such a fluorescent material include SYBR^{®} GREEN I, ethidium bromide, SYTO^{®}-13, SYTO^{®}-16, SYTO^{®}-60, SYTO^{®}-62, SYTO^{®}-64, SYTO^{®}-82, POPO^{®}-3, TOTO^{®}-3, BOBO^{®}-3, TO-PRO^{®}**-**3, YO-PRO^{®}-1, SYTOX Orange^{®}, and the like.

If a fluorochrome molecule is contained only in the droplet at the start of a nucleic acid amplification reaction, the fluorochrome molecule diffuses from the droplet into the droplet encapsulating medium, which makes it difficult to detect an amplified product. Therefore, according to the present invention, the fluorochrome molecule is contained in the droplet encapsulating medium for the purpose of making up for the fluorochrome molecule expected to diffuse.

The fluorochrome molecule may be included only in the droplet encapsulating medium at the start of a nucleic acid amplification reaction. In this case, the fluorochrome molecules initially contained in the droplet encapsulating medium first penetrates the droplet, which makes it possible to detect nucleic acid.

Alternatively, the fluorochrome molecule may be contained in both the droplet and the droplet encapsulating medium at the start of nucleic acid synthesis. The specific concentration of the fluorescent molecule in the droplet and the specific concentration of the fluorescent molecule in the droplet encapsulating medium are not particularly limited. For example, the concentration of the fluorescent molecule in the droplet is sometimes preferably adjusted so as to be higher than that of the fluorescent molecule in the droplet encapsulating medium. This is because a pressure at which the fluorochrome contained in the droplet encapsulating medium penetrates the droplet is high, and therefore the concentration of the fluorochrome in the droplet can be made constant by setting the concentration of the fluorochrome in the droplet encapsulating medium low.

As described above, by allowing the fluorochrome molecule to be contained in at least the droplet encapsulating medium, it is possible to maintain the concentration of the fluorochrome in the droplet at such a level that an amplified product can be stably detected while a nucleic acid amplification reaction keeps going. The method according to the present invention makes it possible to properly maintain the concentration of the fluorochrome in the droplet and therefore to effectively detect an amplified product even at the end of a nucleic acid amplification reaction.

More specifically, the concentration of the fluorochrome contained in the droplet encapsulating medium can be set to 0.01 to 0.5 µM. The upper limit of the concentration may be set to 0.2 µM, 0.1 µM, 0.05 µM, or 0.02 µM. The lower limit of the concentration may be set to 0.02 µM, 0.05 µM, 0.1 µM, or 0.2 µM.
On the other hand, the concentration of the fluorochrome contained in the droplet can be set to 0 to 20 M. The upper limit of the concentration may be set to 10 µM, 5 µM, 2 µM, 1 µM, or 0.5 µM. The lower limit of the concentration may be set to 0.5 µM, 1 µM, 2 µM, 5 µM, or 10 µM. The concentration within the above range is preferred in that it is easy to stably detect an amplified product while a reaction keeps going.
According to the present invention, for example, there is a case where the concentration of the fluorochrome in the droplet encapsulating medium is preferably 0.05 to 0.1 µM, and the concentration of the fluorochrome in the droplet is preferably 0.5 µM to 2 µM.

### [4. Container]

The container is not particularly limited as long as the container can hold the droplet encapsulating medium, and an inner wall of the container has a transport surface on which the droplet is transferred (i.e., with which the droplet is in direct contact). The shape of the container is not particularly limited. For example, the container may comprise a substrate 43 having a transport surface 41 shown in Fig. 4(a); or the container may comprise a bottom member 42 having a transport surface 41 and provided on and in contact with a substrate (ceramic plate) 43, and a wall 44 surrounding the transport surface 41 shown in Fig. 1(b).
The container is provided as one of parts constituting the device according to the present invention, and therefore the device according to the present invention can be provided as a microdevice for droplet manipulation or a chip for droplet manipulation by reducing the size of the container as much as possible.
As shown in Fig. 1(b), the container may further comprise a cover 45 with which a space surrounded by the wall 44 is covered to close the space. The cover 45 may be configured to be fully or partially openable and closable so that a reagent for performing a treatment such as a nucleic acid amplification reaction or a droplet containing a sample can be charged into the container.

From the viewpoint of constructing a perfect closed system, the reaction container is preferably formed by integrally molding a substrate or a bottom member having a transport surface and a wall; or by integrally molding a substrate or a bottom member having a transport surface, a wall, and a cover. Constructing a perfect closed system is very effective because it is possible to prevent contamination with foreign matters during treatment.

### [4-1. Material]

The material of the substrate or the bottom member having a transport surface is not particularly limited, but the transport surface is preferably water repellent to reduce resistance to transfer of the droplet. Examples of a material that imparts such a property include resin materials such as polypropylene, Teflon (Registered Trade Mark), polyethylene, polyvinyl chloride, polystyrene, polycarbonate, and the like. On the other hand, when the container used has a bottom member having a transport surface and provided on a substrate, the substrate may be made of any one of the above-mentioned materials or another material such as ceramic, glass, silicone, or metal.

According to the present invention, the material of the substrate or the bottom member is preferably a resin, particularly preferably polypropylene. When the bottom member is used, a film is preferably used as the bottom member. More specifically, an extra-thin film having a thickness of, for example, 3 µm or less may be used. From the viewpoint of heat resistance required for a reaction or a treatment involving heating, water repellency required during droplet transfer, adhesiveness, processability, and low cost, an extra-thin polypropylene film is preferably used as the bottom member.

Part of the transport surface that is in contact with the droplet and the droplet encapsulating medium may have an affinity for the droplet. For example, such part of the transport surface may be previously subjected to a treatment for relatively reducing water repellency, or a treatment for relatively enhancing hydrophilicity, or a treatment for relatively increasing surface roughness. By placing the droplet in such part of the transport surface, it is possible, even when the droplet encapsulating medium has flowability, to prevent the encapsulated droplet from unintentionally moving.

### [4-2. Physical Properties]

The substrate and the bottom member preferably have light permeability. This makes it possible to perform optical detection when the absorbance of the droplet, fluorescence, chemiluminescence, bioluminescence, or refractive index change is measured from the outside of the reaction container or from the back surface side of the reaction substrate.
Further, the substrate and the bottom member preferably have a surface that can maintain a large contact angle with the droplet even during a reaction or a treatment involving heating. More specifically, polypropylene, or a resin that has a contact angle with the droplet equal to or larger than that of polypropylene with the droplet is preferably used. The contact angle of the droplet on the surface of the substrate is preferably about 95° (deg) to 135° (deg) (at 25°C).

The transport surface that is in contact with the droplet and the droplet encapsulating medium is preferably a smooth surface to transfer the droplet. Particularly, the transport surface preferably has a surface roughness Ra of 0.1 µm or less. For example, when the droplet is transferred by fluctuating a magnetic field by bringing a permanent magnet close to the substrate from the bottom side of the container, the magnetic particles are transferred while being pressed against the surface of the substrate. In this case, by allowing the transport surface to have a surface roughness Ra of 0.1 µm or less, it is possible for the magnetic particles to sufficiently follow the movement of the permanent magnet.

### [4-3. Temperature Variable Region]

The transport surface on which the droplet is transferred has a temperature variable region. The temperature variable region is provided by creating a temperature gradient so that a temperature is continuously changed along a droplet transport path on the transport surface. The temperature gradient is created by, for example, bringing a heat source 5 into contact with part of the bottom surface of the container or part of a substrate 43 shown in Fig. 1(b) which is in contact with the bottom surface of the container, and then heating the heat source 5 at a constant temperature. This makes it possible to provide, on the surface of the substrate or on the surface of the bottom member, a temperature variable region having such a temperature gradient that a temperature is highest at a point located just above the heat source and decreases with the distance from the heat source.

The droplet can be transferred in the temperature variable region by fluctuating a magnetic field and placed at a point having a temperature required for a reaction or a treatment to be performed. The temperature of the liquid constituting the droplet can be quickly adjusted to the temperature of the point simply by transferring the droplet. Therefore, even when a reaction or a treatment to be performed requires a temperature change (e.g., even when a nucleic acid amplification reaction is performed), the temperature of the droplet can be quickly and easily increased and decreased by simply transferring the droplet.

The heat source is set to a temperature highest among temperatures required for a reaction or a treatment to be performed or higher. Further, a cooling source such as a heat sink plate, a cooling fan, or the like may be provided on the low-temperature side of the temperature gradient whose high-temperature side is in contact with the heat source. By providing such a cooling source, it is possible to increase the temperature gradient created in the temperature variable region.
The temperature gradient created in the temperature variable region can be increased also by using a material having low heat conductivity, such as a resin, as a material for the substrate or the bottom member. This makes it possible to perform local temperature adjustment in a narrow region.
By increasing the concentration gradient in this way, it is possible, even when two or more temperature conditions having a relatively large temperature difference are required for a treatment to be performed, to shorten the moving distance of the droplet. This makes it possible to efficiently perform the treatment and reduce the size of the reaction container.

### [5. Magnetic-Field Applying Means]

A magnetic-field applying means or a magnetic-field moving system for fluctuating a magnetic field to transfer the droplet is not particularly limited. As the magnetic-field applying means, a magnetism source such as a permanent magnet (e.g., a ferrite magnet or a neodymium magnet), an electromagnet, or the like can be used. The magnetism source can be provided outside the container in a state where the magnetic particles dispersed in the droplet present in the container can aggregate on the transport surface side. This makes it possible for the magnetism source to apply a magnetic field to the magnetic particles present via the transport surface of the container to capture the aggregated magnetic particles and the droplet containing the magnetic particles.

As the magnetic-field moving system, for example, a system can be used which can move a magnetic field along the transport surface in a state where the magnetic particles can remain aggregated.
For example, as shown in Fig. 4, a system 62 can be used which can mechanically move a magnetism source (e.g., a magnet 61) itself approximately parallel to a transport surface 41. Magnetic particles 8 and a droplet 11 containing the magnetic particles 8 captured via the bottom surface of the container by the magnetism source 61 follow the movement of the magnetism source and therefore can be transferred on the transport surface 41. This makes it possible to transfer the encapsulated droplet, separate a small droplet from the encapsulated droplet regarded as a main (mother) droplet, and coalesce the encapsulated droplet with another encapsulated droplet.

As the magnetic-field moving system, a system that can block or reduce a magnetic field applied to the magnetic particles is also preferably provided. In this case, the system is required to block or reduce a magnetic field to such a degree that the aggregated magnetic particles can be disaggregated and dispersed in the droplet.
For example, an electric current control means can be used. Alternatively, for example, a system can be used which can move a magnet, which is provided via the transport surface outside the container, in a direction approximately perpendicular to the transport surface. In this case, by moving the magnet away from the transport surface, it is possible to block or reduce a magnetic field. This makes it possible to disperse the magnetic particles in the encapsulated droplet to sufficiently expose a component adsorbed to the magnetic particles to the liquid constituting the encapsulated droplet.

Further, a means that can control fluctuations in magnetic field can also be provided. For example, a means which is equipped with a function of vibrating the magnetism source can be used in place of a stirrer. This makes it easy to mix the droplet with another droplet or perform stirring.

As another example of the system that can move a magnetic field along the transport surface, a system that does not involve the above-mentioned mechanical movement of the magnetism source itself may be used. Such a system can be achieved by an array of electromagnets one-dimensionally or two-dimensionally arranged approximately parallel to the transport surface and an electric current control means. In this case, the droplet can be captured by the passage of electric current through the electromagnets and the droplet can be transferred or the magnetic particles can be dispersed by blocking a magnetic field by stopping the flow of electric current through the electromagnets. That is, fluctuations in magnetic field can be controlled by controlling the flow of electric current through the electromagnets. Such an embodiment that does not involve mechanical movement of the magnetism source can be appropriately implemented by those skilled in the art with reference to JP-A-2008-12490.

### [6. Fluorescence Detecting Means]

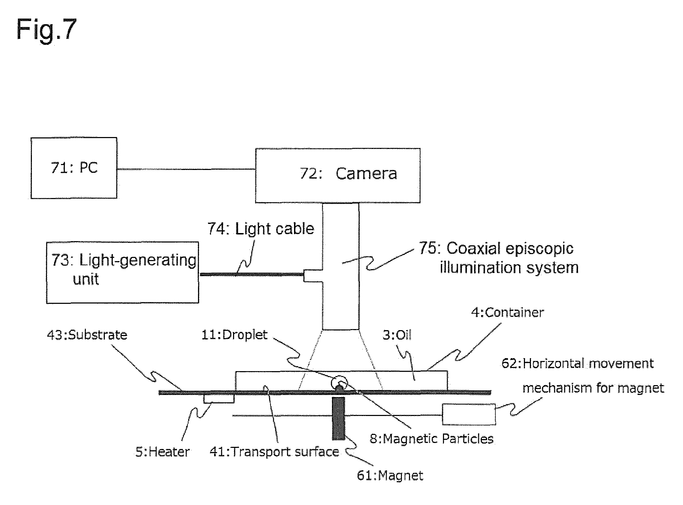

A fluorescence detecting means is not particularly limited and can be easily selected by those skilled in the art. For example, a fluorescence detecting means shown in Fig. 7 comprises a light-generating unit 73, a camera (CCD camera) 72, a coaxial episcopic illumination system 74, and a personal computer (PC) 71. When the fluorescence detecting means is used, light generated by the light-generating unit 73 enters the coaxial episcopic illumination system 75 attached to the CCD camera 72 through a light cable 74 and passes through lenses in the coaxial episcopic illumination system 75 to illuminate a droplet 11 in a reaction container 4. An electric signal detected by the CCD camera is sent to the PC in real time, and therefore a change in the fluorescence intensity of the droplet can be monitored. This is suitable when the present invention is applied to a reaction or a treatment involving detection of changeable fluorescence intensity, such as a real-time nucleic acid amplification reaction.

As the light-generating unit, an LED, a laser, a lamp, or the like can be used. Further, any light-receiving element can be used for detection without any limitation, and examples of such a light-receiving element range from cheap photodiodes to photomultiplier tubes designed for higher sensitivity.
For example, when a nucleic acid-associated reaction such as a real-time nucleic acid amplification reaction or a nucleic acid-associated treatment is performed using, for example, SYBR (Registered Trade Mark) GREEN I, the dye specifically binds to double-stranded DNA and emits fluorescence at about 525 nm, and therefore light can be detected by a light-receiving surface of the CCD camera by cutting off light other than light with an intended wavelength using a filter.
Further, for example, when a nucleic acid amplification reaction is performed, fluorescence emitted from the droplet subjected to the nucleic acid amplification reaction can be observed in a darkroom by irradiating, with exciting light, a point having a temperature at which an extension reaction by DNA polymerase occurs (usually about 68 to 74°C) in a state where the droplet stays at this point. Further, the melting curve of an amplified product can also be obtained and the droplet can be transferred by expanding an area irradiated with exciting light to irradiate an area from a point having a temperature at which thermal denaturation occurs to a point having a temperature at which annealing occurs.

### [7. Manipulation of Droplet and Magnetic Particles]

A droplet encapsulating medium is held in a reaction container so that a droplet can be present in the droplet encapsulating medium. A droplet the entire of which is present in a droplet encapsulating medium is sometimes referred to as an encapsulated droplet.
According to the present invention, droplet manipulation makes it possible to encapsulate a droplet in a droplet encapsulating medium (7-1), transfer an encapsulated droplet (7-2), separate a small droplet from an encapsulated main (mother) droplet (7-3), and coalesce encapsulated droplets to each other (7-4).

It is to be noted that elements required to construct a reaction system or a treatment system to which the present invention is applied may be prepared separately from each other. Such an embodiment is implemented, for example, when it is preferred that an enzyme, a catalyst, or a specific reagent to be subjected to a reaction or a treatment is isolated from another elements until just before the reaction or the treatment to prevent a reduction in activity thereof. An example of this embodiment is one in which elements required to constitute a reaction liquid for nucleic acid amplification are prepared separately from each other. In this case, an enzyme such as a nucleic acid polymerase (e.g., a heat-resistant polymerase used to perform nucleic acid amplification by a hot start method) or a specific reagent for nucleic acid amplification can be isolated from other reagents for nucleic acid amplification until just before the start of reaction.
Another example of this embodiment is one in which a sample to be subjected to a reaction or a treatment is isolated until just before the start of the reaction or the treatment. The sample may be subjected to pretreatment when being isolated. This embodiment is implemented, for example, when nucleic acid to be subjected to an amplification reaction is supplied in the form of a nucleic acid-containing biological sample.

In such cases, a water-based liquid containing one of elements required to construct a reaction system or a treatment system may be placed on a transport path by the above-mentioned method, and a water-based liquid containing the other element may be placed in another position on the transport path by the above-mentioned method. In this case, the isolated one of the elements and the isolated other element can be mixed together by transfer of an encapsulated droplet and coalescence of encapsulated droplets to each other. Alternatively, when separation of a small droplet from an encapsulated main droplet can be performed, the isolated one of the elements and the isolated other element can be mixed together by the separation and coalescence of separated and encapsulated droplets.

Unlike the above-mentioned embodiment, a water-based liquid containing one of elements required to construct a reaction system or a treatment system may be placed on a transport path by the above-mentioned method, while a water-based liquid containing the other element is placed in a droplet state on a gelled droplet encapsulating medium without encapsulating said liquid into a droplet. In this case, the isolated one of the elements and the isolated other element can be mixed together by using an encapsulation method that will be described in 7-1-2.

### [7-1. Encapsulation of Droplet]

### [7-1-1. Method for Encapsulating Droplet by Adding the Droplet]

Droplet encapsulation can be performed by, before the start of droplet manipulation, dissolving a gelling agent in a liquid material contained in a container to prepare a mixed liquid, adding a liquid for forming a droplet to the mixed liquid by dropping or the like, and then, cooling the mixed liquid to turn the liquid into a gel.
Droplet encapsulation can be performed also by, before the start of droplet manipulation, dropping a droplet into a sol-state droplet encapsulating medium, and then, exposing the droplet encapsulating medium to a temperature equal to or lower than sol-gel transition point thereof to turn the medium into a gel; or by directly injecting a water-based liquid into a gel-state droplet encapsulating medium by puncture.

The above methods make it possible to completely encapsulate or fix a droplet in a droplet encapsulating medium. Fixation of a droplet makes storage easy. For example, as shown in Fig. 1(a), encapsulated droplets 12, 13 and 14 may be placed on a transport path so as to come into contact with a transport surface 41 of the inner wall of a container 4.

Droplet encapsulation may be devised in the following manner. For example, as shown in Fig. 4(b), when a droplet encapsulating medium 3 is charged onto a thin bottom member 42 placed on a multi-well device 9 such as a multi-well, the bottom member is bent downward at portions located above the wells by the weight of the encapsulating medium 3 so that recessed portions are formed. By placing droplets 1 at the recessed portions, it is possible, even when the droplet encapsulating medium 3 still has flowability, to prevent the dropped droplets 1 from unintentionally moving. Further, it is also possible, when two or more droplets are encapsulated, to narrow the space between the droplets, which makes it possible to reduce the size of the container.

### [7-1-2. Method for Encapsulating Droplet by Coalescing Droplet on Encapsulating Medium with Encapsulated Droplet]

When a water-based liquid containing one of elements required to construct a reaction system or a treatment system is placed on a transport path by the above-descried method and a water-based liquid containing the other element is placed in a droplet state on a gel-state droplet encapsulating medium, both the elements are mixed together in the following manner.
When a liquid is placed in a droplet state on a gel-state droplet encapsulating medium having no flowability, as shown in, for example, Fig. 1(b), a liquid 2 can be placed in a recess formed in part of the upper surface of a droplet encapsulating medium 31 by pressing or trimming. By forming such a recess, it is possible to prevent the liquid 2 placed on the droplet encapsulating medium 31 from unintentionally spreading or moving. The depth D2 of the recess is not particularly limited. For example, the recess preferably has such a depth that the deepest portion of the recess does not reach a transport surface 41. The recess may have such a depth that the deepest portion of the recess does not reach the highest level of a droplet that has already been encapsulated so as to come into contact with the transport surface 41. More specifically, a depth D2 of about 1 mm is sometimes enough for the recess.

By exposing the droplet encapsulating medium to a temperature equal to or higher than sol-gel transition point thereof, the droplet encapsulating medium is turned into a sol having flowability, and therefore a droplet containing the other element sinks in the droplet encapsulating medium to the bottom surface of a container. The sunken droplet is coalesced with a droplet that contains the one of the elements and has already been encapsulated so that the one of the elements and the other element are mixed together and coexist in one encapsulated droplet. This makes it possible to put the one of the elements and the other element into a state where they can be subjected to a reaction or a treatment.

The droplet containing the other element and the droplet containing the one of the elements can be coalesced together by placing the droplet containing the other element just above the droplet containing the one of the elements that has already been encapsulated. Alternatively, when at least one of the droplet containing the one of the elements and the droplet containing the other element contains magnetic particles, both the droplets can be coalesced together by sinking the droplet containing the other element to the bottom surface of the container so that said droplet is placed in a position different from a position in which the droplet containing the one of the elements has already been encapsulated and then by moving the droplet containing magnetic particles by fluctuating a magnetic field.

Further, as shown in Fig. 2(a), in a state where the droplet encapsulating medium 31 remains gelled, magnetic particles 8 can be separated toward a transport surface 41 while a droplet 2 remains placed on a droplet encapsulating medium 31 by bringing a magnetism source (magnet) 61 close to a container 4 to generate a magnetic field in a direction from the transport surface 41 side to the droplet 2 on the droplet encapsulating medium 31. At this time, the magnetic particles 8 to be separated form an aggregate by magnetism, and the magnetic particles forming an aggregate are separated together with a material adsorbed thereto and a slight amount of liquid adhering to the surfaces thereof. In other words, a small droplet 11b shown in Fig. 21(b) containing the magnetic particles is separated from the droplet 2 shown in Fig. 1(a) regarded as a main droplet. The separated small droplet 11b is guided by the magnetic field and therefore can sink in the droplet encapsulating medium 31 to the transport surface 41 of the container while breaking the three-dimensional structure of the gel (Fig. 2(b)).
In such an embodiment, a specific example of the droplet placed on the droplet encapsulating medium may be a liquid composed of magnetic particles and a sample containing nucleic acid to be amplified. In this case, a small droplet is obtained in a state where the droplet contains the magnetic particles and a liquid composed of the sample containing nucleic acid adsorbed to the magnetic particles.
The sunken small droplet 11b is coalesced with the droplet 14 that contains the one of the elements and has already been encapsulated so that the one of the elements and the other element are mixed together and coexist in one encapsulated droplet 11c. This makes it possible to put the one of the elements and the other element into a state where they can be subjected to a nucleic acid amplification reaction or pretreatment therefor.

### [7-2. Transfer of Encapsulated Droplet]

### [7-2-1. Transfer of Droplet in Sol-State Droplet Encapsulating Medium]

A magnetic particle-containing droplet encapsulated in a sol-state droplet encapsulating medium having flowability is transferred along a droplet transport path on the following principle. As shown in Figs. 2(g) and 2(h), when a magnetic field is generated by bringing a magnet 61 close to a droplet 11g containing magnetic particles in a direction from a transport surface 41 of a container to the inside of the container and is then fluctuated by moving the magnetic field approximately parallel to the transport surface 41 of the container, the magnetic particles are concentrated in the droplet on the side toward which the magnet 61 is moved so that a force trying to transfer the entire droplet in the direction in which the magnet 61 is moved is exerted. As long as traction is transmitted to water constituting the droplet due to the hydrophilic surface of the magnetic particles used in the present invention when the magnetic particles are transferred along the droplet transport surface; and further the contact angle of the droplet on the substrate is sufficiently large; the surface roughness of the transport surface is sufficiently small; the kinetic viscosity of the droplet encapsulating medium is sufficiently small; and the initial velocity of movement of the magnetic field is sufficiently low, it is possible to prevent the magnetic particles from overcoming the surface tension of the droplet and therefore to transfer the entire droplet without allowing the magnetic particles to come out of the droplet.

For example, when 3 µL of magnetic particle dispersion containing magnetic particles having a particle diameter of 3 µm in an amount of 500 µg in water is encapsulated in a droplet encapsulating medium to obtain a droplet, and a ferrite permanent magnet is brought close to the droplet from the outside of a container, under conditions where the contact angle of the droplet on a transport surface is 105° (deg) (at 25°C), the surface roughness Ra of the transport surface is 0.1 µm, and the kinetic viscosity of the droplet encapsulating medium is 15 mm²/s (at 25°C), it is possible to prevent the magnetic particles from overcoming the surface tension of the droplet, that is, it is possible to transfer the entire droplet without allowing the magnetic particles to come out of the droplet as long as the magnet is moved at an initial velocity of 10 cm/sec or less. In this case, it is possible to transfer the entire droplet at a maximum velocity of 100 cm/sec.

Transfer of a droplet containing magnetic particles can be reproducibly performed by setting parameters such as the composition of a water-based liquid constituting the droplet, the particle diameter of the magnetic particles and the amount of the magnetic particles to be used, the contact angel of the droplet on a transport surface, the surface roughness of the transport surface, the kinetic viscosity of a droplet encapsulating medium, the strength of a magnetic field, and the rate at which the magnetic field is fluctuated. Those skilled in the art can adjust each of the parameters by checking the behavior of the magnetic particles contained in the droplet to perform the droplet transfer.
It is to be noted that in this embodiment, the volume of a droplet that can be transferred can be appropriately determined by those skilled in the art. For example, when 10 to 1,000 µg of magnetic particles are used, the volume of a droplet can be set to 0.05 µL to 5 µL.

### [7-2-2. Transfer of Droplet in Gel-State Droplet Encapsulating Medium]

A gel-state droplet encapsulating medium has characteristics inherent to gel, and therefore an encapsulated droplet can be transferred even when the droplet encapsulating medium itself does not have flowability. A magnetic particle-containing droplet encapsulated in a gel-state droplet encapsulating medium can be transferred along a droplet transport path while breaking the three-dimensional structure of gel of the droplet encapsulating medium.

For example, when 3 µL of magnetic particle dispersion containing magnetic particles having a particle diameter of 3 µm in an amount of 500 µg in water is encapsulated in a droplet encapsulating medium to obtain a droplet, and a ferrite permanent magnet is brought close to the droplet from the outside of a container, under conditions where the contact angle of the droplet on a transport surface in the sol-state droplet encapsulating medium is 105° (deg) (at 25°C), the surface roughness Ra of the transport surface is 0.1 µm, and the kinetic viscosity of the gel-state droplet encapsulating medium is 15 mm²/s (at 25°C), it is possible to transfer the entire droplet without allowing the magnetic particles to come out of the droplet as long as the magnet is moved at an initial velocity of 10 cm/sec or less. In this case, it is possible to transfer the entire droplet at a maximum velocity of 100 cm/sec.

In an embodiment in which a droplet is transferred in a gel-state droplet encapsulating medium, the volume of the droplet that is carried by magnetic particles is often as small as the volume of the droplet adhering to the surfaces of magnetic particles. For example, when magnetic particles are used in an amount of 100 to 500 µg, the volume of a droplet that is carried by the magnetic particles is only about 1 µL to 5 µL. This embodiment is suitable when the amount of a droplet carried together with magnetic particles is preferably as small as possible, such as when an intended component to be carried by magnetic particles is only the component adsorbed to the surfaces of the magnetic particles.

### [7-2-3. Transfer on Temperature Variable Region]

As mentioned above, the embodiment in which an encapsulated droplet itself is transferred is preferably used when the liquid temperature of the encapsulated droplet needs to be changed. When a transport surface has a temperature variable region provided by creating a temperature gradient along a droplet transport path, the liquid temperature of an encapsulated droplet can be quickly and easily adjusted by transferring the encapsulated droplet itself to a point having a temperature required for treatment performed in a liquid constituting the encapsulated droplet.

Therefore, the present invention is useful, for example, when a nucleic acid amplification reaction requiring two or more temperature conditions having a relatively large difference is performed. For example, among the above-mentioned methods for nucleic acid amplification reaction, a PCR method, a LCR method, a TAS method, and the like are required to repeat a thermal cycle requiring two or three temperature conditions having a relatively large difference multiple times. According to the method of the present invention, as shown in Figs. 2 (g) and 2(h), an encapsulated droplet 11g composed of a reaction liquid for nucleic acid amplification containing the above-mentioned magnetic particles having hydrophilic surfaces, nucleic acid to be amplified, fluorochrome, and materials required for nucleic acid amplification reaction is transferred to a point having a temperature required for performing each of the steps of a nucleic acid amplification reaction by applying a fluctuating magnetic field to the droplet, and is allowed to stay at each of the point for necessary time. Therefore, complicated temperature conditions required for a nucleic acid amplification reaction can be easily achieved. Further, an amplified product can be appropriately detected by allowing a fluorochrome to be contained in at least a droplet encapsulating medium, which makes it possible to observe a nucleic acid amplification reaction performed in the droplet in real time (real-time nucleic acid amplification).

Further, the present invention can be flexibly applied to a reaction or a treatment that may be selected by a user even when the reaction or treatment requires a wide range of temperature conditions. For example, an SDA method, a Qβ method, a NASBA method, an ICAN method, an ICAT method, an RCA method, and the like are methods for isothermal amplification reaction performed under one temperature condition in the range of about 37°C to 65°C, but an optimum temperature differs depending on an object to be amplified. When the method according to the present invention is applied to any one of these nucleic acid amplification methods, desirable amplification efficiency can be achieved by simply placing a droplet at a point where the temperature of the droplet can be controlled at an optimum temperature for an object to be amplified.

### [7-3. Separation of Magnetic Particles and Small Droplet Attached thereto from Encapsulated Main Droplet]

### [7-3-1. Separation of Small droplet in Sol-State Droplet Encapsulating Medium]

A modification of the above-mentioned embodiment in which a droplet is transferred in a sol-state droplet encapsulating medium is embodiment in which the encapsulated droplet to be transferred is a small droplet separated from an another droplet regarded as a main (mother) droplet.
The another droplet is one encapsulated in the droplet encapsulating medium in the same container. In this embodiment, a magnetic field is applied to magnetic particles contained in the encapsulated another droplet to transfer the magnetic particles along a transport path so that the aggregated magnetic particles are drawn out of and separated from the main droplet without transferring the entire encapsulated main droplet. At this time, the separated aggregated magnetic particles convey around the surfaces thereof a material adsorbed thereto and a small amount of liquid (small droplet) derived from the main droplet.

For example, magnetic particles and a small droplet adhering thereto can be separated from a main droplet containing the magnetic particles by changing the above-mentioned various conditions allowing droplet transfer so that the amount of the magnetic particles contained is made relatively smaller in respect to a main droplet; the contact angle of the droplet on a transport surface is made relatively smaller; the surface roughness of the transport surface is made relatively larger; the kinetic viscosity of a droplet encapsulating medium is made relatively higher; or the initial velocity of fluctuation of a magnetic field is made relatively higher compared to each of the conditions for the droplet transfer. By significantly changing the conditions described above as examples, it is possible to increase the volume of the small droplet adhering to the magnetic particles. As in the case of the above-mentioned droplet transfer, separation of a small droplet can be performed by those skilled in the art by adjusting each of the parameters by checking the behavior of the magnetic particles contained in the droplet.

In this embodiment, the droplet encapsulating medium is in a sol-state and has flowability, and therefore the encapsulated main droplet itself is not fixed. For this reason, the droplet is more easily moved in the droplet encapsulating medium when the above-mentioned conditions are closer to the conditions for the transfer of the droplet itself, which tends to make it difficult to separate the magnetic particles and a small droplet adhering thereto from the main droplet. In this case, for example, a spot having an affinity for the droplet may be provided in part of the transport path on the transport surface. For example, by previously subjecting the spot to a treatment for relatively reducing water repellency, relatively increasing hydrophilicity, or relatively increasing surface roughness, it is possible to prevent the main droplet placed on the spot from unintentionally moving. Further, the similar effect can be obtained also by controlling an electric field by, for example, separately applying an unmoving magnetic field to the encapsulated main droplet in a desired position on a substrate from the bottom side of the substrate.

### [7-3-2. Separation of Small Droplet in Gel-State Droplet Encapsulating Medium]

On the other hand, as shown in Figs. 2(c) to 2(e), magnetic particles and a small droplet 11e adhering thereto can be separated also from a main droplet 11c containing the magnetic particles and encapsulated in a gel-state droplet encapsulating medium 31 while the droplet encapsulating medium 31 remains in a gel state having no flowability. This embodiment is based on the same principle as the embodiment shown in Figs. 2(a) to 2(b) in which the magnetic particles and the small droplet 11b adhering thereto are separated from the main droplet 2 containing the magnetic particles and placed on the droplet encapsulating medium 31.

More specifically, the magnetic particles to be separated form an aggregate by magnetism, and the aggregated magnetic particles are separated together with a material adhered thereto and a small amount of liquid (Fig. 2(e)). In other words, the small droplet 11e containing the magnetic particles is separated from the encapsulated droplet 11c regarded as a main droplet. The separated small droplet 11e can be transferred along a transport path while breaking the three-dimensional structure of gel of the droplet encapsulating medium 31 under the guidance of a magnetic field. On the other hand, the encapsulated droplet whose volume is larger by a certain degree than the aggregated magnetic particles (i.e., the main droplet 11c) is fixed by the gel-state encapsulating medium and therefore cannot be displaced together with the aggregated magnetic particles. Therefore, the magnetic particles 8 are separated together with the small droplet 11e adhering thereto, but the main droplet stays in its initial position (Figs. 2 (d) and 2(e)). This makes it possible to very easily separate a small droplet containing magnetic particles from a main droplet without using a method (e.g., electric-field control) which may be used in the above-mentioned case using a droplet encapsulating medium having flowability to prevent an encapsulated droplet from unintentionally moving. For this reason, a gel-state droplet encapsulating medium has a very high degree of flexibility in the placement of a droplet, which makes it possible to flexibly determine a droplet transport path.

Further, as has been already described, in the embodiment in which a droplet is transferred in a gel-state droplet encapsulating medium, the volume of the droplet carried by magnetic particles is often as very small as that of the droplet adhering to the surfaces of the magnetic particles. Therefore, when a desired component to be carried by the magnetic particles is only the component adsorbed to the surfaces of the magnetic particles, the embodiment in which a small droplet is separated from a main droplet in a gel-state droplet encapsulating medium is preferred from the viewpoint of minimizing the amounts of extra liquid components carried by the magnetic particles to accurately separate the component adsorbed to the magnetic particles.

### [7-4. Coalescence of Encapsulated Droplet and Encapsulated Droplet Containing Magnetic Particles]

A droplet containing magnetic particles can be coalesced with an another encapsulated droplet in the same container by exposure to a liquid constituting the another encapsulated droplet. A droplet encapsulating medium in which the another encapsulated droplet is encapsulated may be either in a gel state or in a sol state. By coalescing droplets together, mixing of components constituting the droplets, dissolution, or dilution can be performed.
For example, when the present invention is applied to a reaction performed by mixing two or more reagents, the reaction can be performed by transferring an encapsulated droplet containing one of the reagents and magnetic particles and coalescing said droplet with an encapsulated droplet containing the other reagent (and further, when a transport surface has a temperature variable region, by transferring a droplet obtained by coalescing the encapsulated droplets to a point having a temperature suitable for the reaction). The thus obtained reaction product can be further reacted with another reagent in the same manner.

Further, when the present invention is applied to a nucleic acid-related treatment or reaction, a small droplet separated from an encapsulated main droplet composed of a liquid containing nucleic acid and magnetic particles by applying a fluctuating magnetic field can be coalesced with an another encapsulated droplet composed of a liquid in which a treatment such as a nucleic acid amplification reaction is performed. The another encapsulated droplet is, for example, a liquid composed of a nucleic acid extraction liquid, a liquid composed of a cleaning liquid, a liquid composed of a nucleic acid releasing liquid or the like.

For example, when a treatment for extracting nucleic acid is performed, a nucleic acid component contained in a small droplet 11b can be extracted by transferring the small droplet 11b containing magnetic particles and nucleic acid and other components adhering thereto in a droplet encapsulating medium 31 as shown in Fig. 2(b), and then coalescing the small droplet 11b with an another encapsulated droplet 14 composed of a nucleic acid extraction liquid (Fig. 2(c)). Further, as shown in Figs. 2(d) and 2(e), by applying a fluctuating magnetic field, the magnetic particles are separated together with the extracted nucleic acid and a small droplet 11e adhering thereto from an encapsulated droplet 11c composed of the nucleic acid extraction liquid coalesced with the small droplet 11b, and are transferred in the droplet encapsulating medium 31.

A treatment for cleaning the magnetic particles can also be performed in the same manner. That is, the magnetic particles can be cleaned by transferring the another small droplet containing the magnetic particles and the nucleic acid adhering thereto in the encapsulating medium, and then coalescing the small droplet with an another encapsulated droplet composed of a cleaning liquid. By cleaning the magnetic particles, the nucleic acid adsorbed to the magnetic particles can be cleaned. Further, by applying a fluctuating magnetic field, the magnetic particles are separated together with the cleaned nucleic acid and a small droplet adhering thereto from the encapsulated droplet composed of the cleaning liquid, and are transferred in the encapsulating medium. A treatment for releasing the nucleic acid is also performed in the same manner.

A nucleic acid-containing sample or a small droplet that has been subjected to the above-mentioned nucleic acid extraction treatment, cleaning treatment, and/or nucleic acid releasing treatment if necessary is coalesced with a droplet composed of a reaction liquid for nucleic acid amplification (Figs. 2(f) and 2(g)). This makes it possible to obtain a droplet 11g composed of the reaction liquid for nucleic acid amplification containing nucleic acid to be amplified and magnetic particles. A nucleic acid amplification reaction can be initiated by transferring the obtained droplet 11g to a point in the temperature variable region having a temperature at which a nucleic acid amplification reaction occurs (Fig. 2(h)).
As described above, a series of treatments including a nucleic acid amplification reaction and pretreatment therefor is performed in a perfect closed system. Further, these treatments can be easily performed by dispersing magnetic particles in an encapsulated droplet, aggregating the magnetic particles for transfer, and transferring the magnetic particles between droplets and between points having desired temperatures in a gel.

### EXAMPLES

The present invention will be described in more detail with reference to the following examples.

### [Example 1]

As a nucleic acid-containing sample, a mixed liquid of an oral swab sample liquid and a cell lysate was used. The oral swab sample liquid was prepared by suspending oral mucosal cells scraped using a cotton swab in 1 mL of distilled water. By mixing 25 µL of the oral swab sample liquid and a cell lysate containing guanidine thiocyanate in a final concentration of 2 M, 50 µL of a mixed liquid was prepared.
A cleaning liquid was prepared as a mixed solution of 200 mM potassium chloride and 50 mM Tris-HCl (pH 8.0).

A reaction liquid for PCR was prepared as a mixed liquid containing 0.125 U TaqDNA polymerase (manufactured by TAKARA BIO INC.), primers for β-actin detection, each at a concentration of 500 nM, 500 µM dNTP, 10 mM magnesium chloride, 10 mM Tris-HCl buffer (pH 9.2), and 0.2 weight % bovine serum albumin (manufactured by SIGMA). It is to be noted that one of the primers for β-actin detection has a sequence of 5'-TGGCATCGTGATGGACTCCGGTGA-3' (SEQ ID No. 1) and the other primer for β-actin detection has a sequence of 5'-GCTGTAGCCGCGCTCGGTGAGGAT-3' (SEQ ID No. 2).

As a container, one having a polycarbonate frame and a 2.8 µm-thick polypropylene film used as a bottom member was used.
A droplet encapsulating medium material was prepared by adding 12-hydroxystearic acid (manufactured by Wako Pure Chemical Industries Ltd.) to silicone oil (Shin-Etsu Silicone KF-56) so that the concentration of 12-hydroxystearic acid was 1 weight %, and then heating the mixture to 90°C so that the 12-hydroxystearic acid was completely mixed with the silicone oil. The mixed oil was filled into the container so that the thickness (filling height) of the oil layer was 3 mm. Then, the temperature of the oil was lowered to about 60°C, and 3 droplets of 20 µL of the cleaning liquid and 1 droplet of 1 µL of the reaction liquid for PCR were placed in the oil as shown in Figs. 1(a) and 1(b). The oil was allowed to stand until it was cooled to a room temperature so that the entire oil was turned into a gel.

As shown in Fig. 1(b), a recess of about 1 mm was formed in part of the surface of the gelled oil, and 50 µL of the cell lysate and the oral swab sample liquid containing 100 µg of magnetic silica particles (magnetic beads MagExtractor -Genome- manufactured by TOYOBO Co., Ltd.) was placed in the recess. The container was covered with a 3 mm-thick polycarbonate plate.

As shown in Fig. 1(b), the end of a 1 mm-thick alumina ceramic plate was heated by an electric heater, and then the container was placed on the ceramic plate at the time when a stable temperature gradient was created on the surface of the ceramic plate, and was further allowed to stand for 10 minutes to allow the oil in the container to have the same temperature gradient. After the container was allowed to stand, the oil in the container had both a part that was turned into a sol state by the temperature gradient created on the ceramic plate and a part that remained in a gel state. The oil present in a high-temperature side region including a region where the reaction liquid for PCR was surrounded by the oil was turned into a sol, and the oil present in a low-temperature side region where the cleaning liquid was surrounded by the oil was in a sol-state. The oil used in this example had a gel-sol transition temperature of about 50°C.

As shown in Figs. 2(a) to 2(h), droplet manipulation was performed using a magnet. In this example, a method for isolating nucleic acid using silica particles and a chaotropic salt (JP-A-2-289596) was used.
First, as shown in Figs. 2 (a) and 2(b), an aggregate of the magnetic particles was separated from a droplet composed of the mixed liquid of the cell lysate and the oral swab sample containing the magnetic silica particles and moved into the lower oil layer, by vertically and upwardly moving the magnet at a rate of 2 mm/sec to bring the magnet close to the ceramic plate. Guanidine thiocyanate contained in the mixed liquid lysed oral mucosal cells contained in the oral swab so that released nucleic acid was adsorbed to the surfaces of the magnetic particles.

As shown in Figs. 2(b) and 2(c), the magnet was moved toward the high-temperature side to clean the magnetic silica particles with the first cleaning liquid. This cleaning was performed to remove sample-derived components, other than nucleic acid, inhibiting a PCR reaction and guanidine thiocyanate from the aggregate of the magnetic silica particles. Further, as shown in Figs. 2(c) to 2(f), the magnet was moved toward the high-temperature side to allow the magnetic silica particles to pass through the three droplets of the cleaning liquid. Then, as shown in Fig. 2(g), the aggregate of the magnetic silica particles was coalesced with the droplet composed of the reaction liquid for PCR. the oil surrounding the droplet composed of the reaction liquid for PCR is close to the heater and has a high temperature, and is therefore turned into a sol. Therefore, the reaction liquid for PCR itself containing the magnetic particles can be transferred together with the magnetic particles by manipulating the magnet. The aggregate of the magnetic silica particles separated from the last cleaning liquid includes small amounts of potassium chloride and Tris-HCL buffer (pH 8.0) derived from the cleaning liquid, but these components do not significantly inhibit an enzymatic reaction.

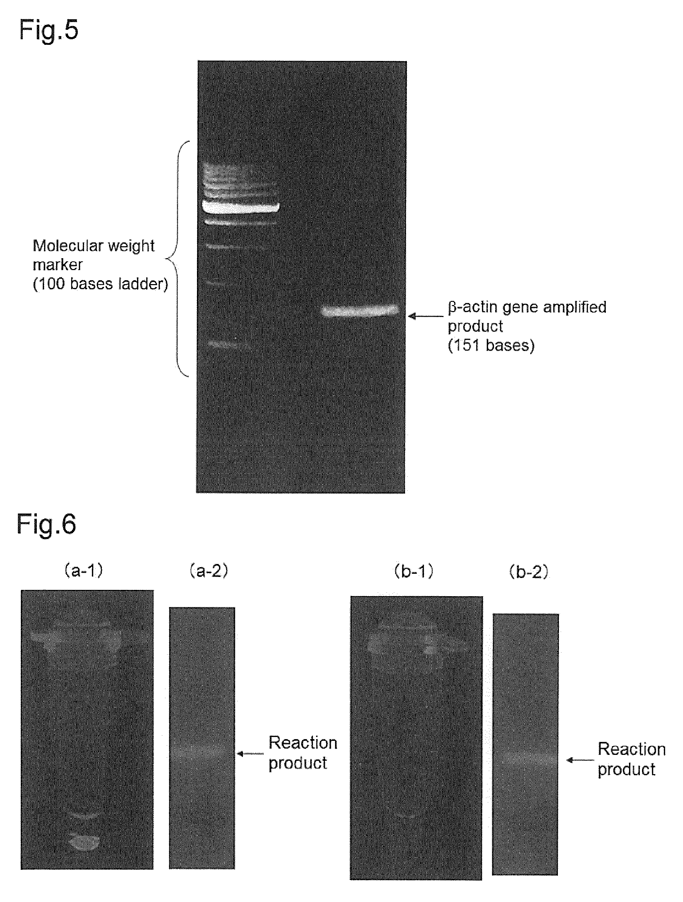

As shown in Fig. 2(h), the obtained droplet shown in Fig. 2 (g) was transferred by the magnet on the ceramic plate having a temperature gradient (more specifically, a temperature gradient from 94°C to 60°C). At this time, the droplet was reciprocated (40 cycles) by moving the magnet based on a thermal program including at 94°C for 2 seconds, at 60°C for 1 second, and at 72°C for 5 seconds to complete a PCR reaction.

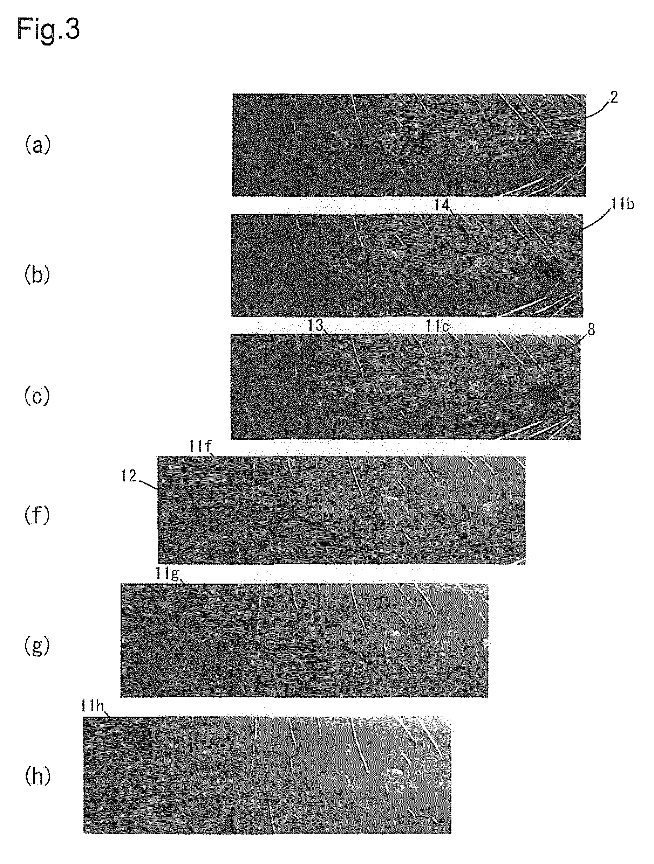

Fig. 3 shows photographs taken during the above-mentioned series of operations performed in this example. Symbols (a) to (h) and numerals attached to elements in the photographs shown in Fig. 3 correspond respectively to those shown in Fig. 2.
After the completion of the reaction, the droplet was subjected to agarose-gel electrophoresis. As a result, an amplified product (151 bases) from a β-actin gene was identified (Fig. 5).

As described above, separation of a specimen, cleaning, extraction of nucleic acid from the specimen, and amplification of a target gene by PCR by magnetic particles could be performed on one transport surface in one container without providing a physical fluid control system in the container simply by transferring the magnetic particles and a droplet composed of a reaction liquid for PCR on the transport surface (on the surface of a bottom member) by using a magnet.

In the following Reference Examples 1 and 2, a nucleic acid amplification reaction was performed in a state where a fluorochrome was contained in at least a droplet encapsulating medium. As the droplet encapsulating medium, a non-gelled droplet encapsulating medium (that is, a droplet encapsulating medium containing no gelling agent) was used. The present inventors have already confirmed that the same results as in the following reference examples can be obtained also when the gel-state droplet encapsulating medium according to the present invention is used.

### [Reference Example 1]

As magnetic particles having hydrophilic surfaces, Magnetic Beads included as a constituent reagent in Plasmid DNA Purification Kit MagExtractor -Genome- kit available from TOYOBO Co., Ltd. (hereinafter, simply referred to as "magnetic silica beads") were used. The magnetic silica beads included in the kit were previously cleaned by repeating the following operation five times: the magnetic silica beads were suspended in pure water whose volume was ten times larger than that of an undiluted liquid containing the magnetic silica beads, and then the suspension was centrifuged at 500×g for 1 minute to remove supernatant. Then, the magnetic silica beads were suspended in pure water so that the amount of the magnetic silica beads contained in the pure water was adjusted to 100 mg (dry) /mL in terms of dry weight of the beads.

The composition of a reaction liquid for PCR was as follows: 50 mM potassium chloride, 10 mM Tris-HCl buffer (pH 9.5), 5 mM magnesium chloride, 0.6 µM PCR primer for β-actin detection (Forward) (manufactured by Applied Biosystems), 0.6 µM PCR primer for β-actin detection (Reverse) (manufactured by Applied Biosystems), and 0.75 U heat-resistant DNA polymerase (Ex Taq DNA Polymerase manufactured by TAKARA SHUZO CO., LTD.) . Further, in order to prevent deactivation of the DNA polymerase caused by adsorption to the surface of a substrate, the magnetic particles, the interface with oil, etc., 0.1 weight % bovine serum albumin was added. To the reaction liquid for PCR were added 3 ng of purified standard human genomic DNA (manufactured by Roche) and the magnetic silica beads so that the concentration of the magnetic silica beads was 10 µg/µL in terms of dry weight of the beads.

As a bottom member of a reaction container, a 2.8 µm-thick polypropylene film (ALPHAN EM-501K manufactured by Oji Specialty Paper Co., Ltd.) was used, and a silicone oil (KF-56 manufactured by Shin-Etsu Chemical Co., Ltd.) was filled into the reaction container.
SYBR^{®} GREEN I manufactured by Invitrogen was added to a droplet composed of the reaction liquid for PCR so as to be diluted to a concentration 10,000 times smaller than that of its undiluted liquid product. Further, SYBR^{®} GREEN I was added to the silicone oil so as to be diluted to a concentration 50, 000 times smaller than that of its undiluted liquid product.

When a gene amplified product is produced, fluorescence emitted when the fluorochrome binds to double-stranded DNA is observed. The results of a PCR reaction performed according to this reference example are shown in Fig. 6. Fig. 6(a-1) is an image obtained by observing fluorescence of SYBR^{®} GREEN I by ultraviolet irradiation after the completion of PCR when the fluorochrome was added to the silicone oil and Fig. 6(b-1) is an image obtained by observing fluorescence of SYBR^{®} GREEN I by ultraviolet irradiation after the completion of PCR when the fluorochrome was not added to the silicone oil. Only when the fluorochrome was added to the silicone oil (a-1), a signal was observed from the droplet collected in a polypropylene tube by ultraviolet irradiation. This signal was observed as yellow-green fluorescence having a wavelength of 472 nm derived from SYBR^{®} GREEN I. On the other hand, gene amplification occurred also in (b-1), but fluorescence was hardly observed.

It is to be noted that the results of agarose-gel electrophoresis of the gene amplified products obtained in (a-1) and (b-1) are shown in Figs. 6(a-2) and 6(b-2), respectively. As shown in Figs. 6(a-2) and 6(b-2), in both cases, the gene amplification reaction was normally completed.

### [Reference Example 2]

PCR was performed in the same manner as in Reference Example 1 except that each of fluorochromes, SYBR-Green I, YO PRO-1, and SYTO-13 (all of which are manufactured by Invitrogen) were used and that the concentration of each of the fluorochromes contained in the droplet and the concentration of each of the fluorochromes contained in the oil were varied. Differences between the intensity of fluorescence observed before the start of PCR and the intensity of fluorescence observed after the start of PCR are shown in Tables 1 to 3. Table 1 shows results obtained using SYBR-Green I, Table 2 shows results obtained using YO PRO-1, and Table 3 shows results obtained using SYTO-13.

It is to be noted that all the droplets had a volume of 3 µL, and the composition of the reaction liquid was as follows: 25 mM Tris-HCl (pH 8.3), 8 mM MgCl₂, 0.2% (w/v) bovine serum albumin, 0.125 U/µL Ex Taq DNA polymerase (manufactured by TAKARA BIO INC.), 250 µM dNTP, and primers for human β-actin gene detection (each 1 µM).
One of the primers for human β-actin gene detection has a sequence of 5'-CATCGAGCACGGCATCGTCACCAA-3' (SEQ ID No. 1) and the other primer for human β-actin gene detection has a sequence of 5'-GCGGGCCACTCACCTGGGTCATCT-3' (SEQ ID No. 2).

To the droplet of 3 µL, 510 µg of magnetic beads (MagExtractor (R) -Plasmid- manufactured by TOYOBO Co., Ltd.) were added. As a droplet encapsulating medium, a silicone oil KF-56 manufactured by Shin-Etsu Chemical Co. Ltd. was used. PCR was performed under conditions described in T. Ohashi, H. Kuyama, N. Hanafusa, and Y. Togawa: Biomed. Microdevices, 9, 695 (2007). More specifically, one PCR cycle consisting of thermal denaturation (95°C, 0.5 sec), annealing (60°C, 1 sec), and extension (72°C, 5 sec) was repeated 40 times in total. The PCR cycle was performed by transferring the droplet composed of the reaction liquid containing the magnetic beads by moving the magnet provided outside the container and located just below the droplet at a rate of 1.1 cm/sec between a spot having a temperature of 95°C and a spot having a temperature of 60°C provided by creating a temperature gradient.

The fluorescence intensity of the droplet was measured using a cooled CCD camera (ST-402ME manufactured by SBIG) by taking an image from directly above the droplet in the oil with exposure for 5 seconds at maximum sensitivity. An exciting light source was a 470 nm blue LED, an exciting light-side band-pass filter was a 475nm/40nm band-pass filter, and a detection-side band-pass filter was a 535nm/45nm band-pass filter. Image analysis software Image J was used to calculate the amount of fluorescence of the entire droplet as a relative fluorescence intensity, and a value obtained by subtracting a fluorescence intensity measured before PCR (i.e., background) from a fluorescence intensity measured after PCR was defined as a data value. It has been found that, in this reference example, the optimum concentration of each of the fluorochromes in the droplet is in the range of about 0. 5 to 2 µM and the optimum concentration of each of the fluorochromes in the oil is in the range of about 0.05 to 0.1 µM. When the fluorochrome was not previously added to the oil, a significant increase in fluorescence intensity was not detected. On the other hand, it has been found that fluorescence of amplified nucleic acid can be detected without adding the fluorochrome to the droplet as long as the fluorochrome is previously present in at least the oil.

**[Table 1]**

| | **SYBR Green I** | | Concentration of Fluolochrome in Droplet (µm) | | | | | |
|---|---|---|---|---|---|---|---|---|
| | | 0 | 0.5 | 1 | 2 | 5 | 10 | 20 |
| Concentration of Fluolochrome in Oil (µM) | 0 | 0 | -18 | -34 | -89 | -145 | -278 | -450 |
| | 0.01 | 46 | 32 | -9 | -25 | -89 | -123 | -241 |
| | 0.02 | 78 | 90 | 35 | -6 | -34 | -89 | -178 |
| | 0.05 | 156 | 178 | 202 | 78 | 12 | -10 | -66 |
| | 0.1 | 267 | 345 | 356 | 207 | 67 | 20 | -33 |
| | 0.2 | 176 | 150 | 89 | 67 | 34 | 22 | 17 |
| | 0.5 | 124 | 103 | 60 | 23 | 9 | -22 | -7 |

| | | | | | | | | |
|---|---|---|---|---|---|---|---|---|
| Data Value Unit: RFU (Relative fluorescent Unit) | | | | | | | | |

**[Table 2]**

| | **YO PRO-1** | | Concentration of Fluolochrome in Droplet (µM) | | | | | |
|---|---|---|---|---|---|---|---|---|
| | | 0 | 0.5 | 1 | 2 | 5 | 10 | 20 |
| Concentration of Fluolochrome in Oil (µM) | 0 | 0 | 7 | 11 | 4 | -23 | -189 | -356 |
| | 0.01 | 70 | 123 | 167 | 203 | 170 | -45 | -177 |
| | 0.02 | 127 | 234 | 321 | 345 | 124 | -21 | -123 |
| | 0.05 | 280 | 340 | 450 | 521 | 278 | 29 | -78 |
| | 0.1 | 329 | 452 | 389 | 179 | 88 | -19 | -23 |
| | 0.2 | 256 | 498 | 367 | 98 | 7 | -7 | -6 |
| | 0.5 | 224 | 309 | 51 | 18 | -5 | 0 | -3 |

| | | | | | | | | |
|---|---|---|---|---|---|---|---|---|
| Data Value Unit: RFU (Relative fluorescent Unit) | | | | | | | | |

**[Table 3]**

| | **SYTO-13** | | Concentration of Fluolochrome in Droplet (µM) | | | | | |
|---|---|---|---|---|---|---|---|---|
| | | 0 | 0.5 | 1 | 2 | 5 | 10 | 20 |
| Concentration of Fluolochrome in Oil (µM) | 0 | 0 | -13 | -24 | -45 | -89 | -135 | -240 |
| | 0.01 | 45 | 34 | 37 | 67 | 37 | -50 | -169 |
| | 0.02 | 67 | 55 | 65 | 91 | 67 | 6 | -89 |
| | 0.05 | 89 | 80 | 103 | 85 | 67 | 13 | -16 |
| | 0.1 | 82 | 56 | 45 | 67 | 40 | -5 | 9 |
| | 0.2 | 67 | 34 | 39 | 30 | 19 | 2 | -6 |
| | 0.5 | 46 | 24 | 30 | 17 | 7 | -2 | 1 |

| | | | | | | | | |
|---|---|---|---|---|---|---|---|---|
| Data Value Unit: RFU (relative fluorescent Unit) | | | | | | | | |

Although the present invention has been described above with reference to the above embodiments, the description and the drawings that constitute part of the disclosure should not be construed as limiting the present invention. Various alternative embodiments, examples, and practical applications will be apparent from the disclosure to those skilled in the art. The technical scope of the present invention is defined only by the invention-specifying matters according to the scope of claims reasonable from the above description. The present invention can be modified in various ways without departing from the scope of the invention.

### SEQUENCE LISTING FREE TEXT

SEQ ID No. 1: synthetic primer
SEQ ID No. 2: synthetic primer

## Claims

1. A droplet manipulation device for transporting a droplet in a droplet encapsulating medium, comprising:
a container which holds the droplet encapsulating medium;
a droplet composed of a water-based liquid;
a gel-state droplet encapsulating medium which is insoluble or poorly soluble in the water-based liquid;
magnetic particles included in the droplet composed of the water-based liquid; and
means for applying a magnetic field to generate a magnetic field to transport the droplet together with the magnetic particles.

2. The droplet manipulation device according to claim 1, wherein the gel-state droplet encapsulating medium is prepared by mixing a water-insoluble or poorly water-soluble liquid material, and a gelling agent selected from the group consisting of hydroxy fatty acids, dextrin fatty acid esters, and glycerin fatty acid esters.

3. The device according to claim 1, wherein an another droplet is placed in a path for transporting the droplet.

4. The device according to claim 1, wherein the path for transporting the droplet has a temperature gradient.

5. A method for manipulating a droplet in a droplet encapsulating medium,
wherein the droplet encapsulating medium is held in a container,
the droplet is composed of a water-based liquid including magnetic particles, and
the droplet encapsulating medium is in a gel state at least before start of droplet manipulation, and is insoluble or poorly soluble in the water-based liquid when the medium is in gel and sol states;
the method comprising the step of, during the droplet manipulation, transporting the droplet together with the magnetic particles by generating a magnetic field by means for applying a magnetic field.

6. The method according to claim 5, wherein, before start of the droplet manipulation, a container containing a mixture of a water-insoluble or poorly water-soluble liquid material and a gelling agent is prepared, a droplet is added to the mixture, and then the mixture is turned into a gel to encapsulate the droplet in a gel-state droplet encapsulating medium.

7. The method according to claim 5, wherein the droplet is one separated from an another droplet, which includes the magnetic particles and is encapsulated in the gel- or sol-state droplet encapsulating medium in a path for transporting the droplet in the same container, by applying the magnetic field to the another droplet and transferring the droplet along the path for transporting the droplet.

8. The method according to claim 5, wherein the droplet is one separated from an another droplet, which includes the magnetic particles and is placed on the gel-state droplet encapsulating medium in the same container, by generating the magnetic field to the another droplet.

9. The method according to claim 5, wherein the droplet is transferred in the gel- or sol-state droplet encapsulating medium and thereby is coalesced with an another droplet encapsulated in the droplet encapsulating medium in a path for transporting the droplet in the same container.

10. The method according to claim 5, wherein a path for transporting the droplet has a temperature gradient.

11. The method according to claim 10, wherein the droplet encapsulating medium has, in the same container, both a sol phase formed on a high-temperature side of the temperature gradient and a gel phase formed on a low-temperature side of the temperature gradient.

12. The method according to claim 9, wherein the another droplet is composed of a cleaning liquid and the magnetic particles and a component adsorbed thereto are cleaned by the coalescence.

13. The method according to claim 8, wherein a cell lysate and a biological sample are contained in the another droplet to adsorb nucleic acid derived from the biological sample to the magnetic particles.

14. The method according to claim 11, wherein the another droplet is composed of a nucleic acid amplification reaction liquid, and
wherein, in the sol-state droplet encapsulating medium, a droplet composed of a reaction mixture obtained by the coalescence is transferred to a point, which is located on the path for transporting the droplet having the temperature gradient and has a temperature at which a nucleic acid synthesis reaction starts and keeps going, to control a temperature of the reaction mixture.

15. The method according to claim 14, wherein at start of the nucleic acid synthesis reaction, a fluorochrome is included in at least the droplet encapsulating medium out of the droplet composed of the reaction mixture and the droplet encapsulating medium.

16. A kit for preparing the device according to claim 1, comprising:
a container which holds the droplet encapsulating medium;
the gel-state droplet encapsulating medium, or a water-insoluble or poorly water-soluble liquid material and a gelling agent which are materials for preparing the gel-state droplet encapsulating medium;
magnetic particles; and
means for applying a magnetic field.
